# FASCICULE DE BREVET EUROPEEN

(11) **EP 1 051 144 B1**
(45) Date de publication et mention de la délivrance du brevet: **03.09.2003**
(21) Numéro de dépôt: 99900944.2
(22) Date de dépôt: 18.01.1999
(51) Int. Cl.: A61K 7/06, A61K 7/09, A61K 7/13

(54) **UTILISATION A TITRE D'AGENT PROTECTEUR DES FIBRES KERATINIQUES DE POLYMERES DE POLYAMMONIUM QUATERNAIRE HETEROCYCLIQUE ET COMPOSITIONS COSMETIQUES**
VERWENDUNG VON QUATERNÄREN HETEROZYKLISCHEN POLYAMMONIUMPOLYMEREN ALS SCHUTZMITTEL DER KERATINISCHEN FASER UND KOSMETISCHE ZUSAMMENSETZUNGEN
USE OF HETEROCYCLIC QUATERNARY POLYAMMONIUM POLYMERS AS PROTECTIVE AGENT FOR KERATIN FIBRES AND COSMETIC COMPOSITIONS

(30) Priorité: 26.01.1998 FR 9800793
(43) Date de publication de la demande: 15.11.2000
(73) Titulaire: L'OREAL, 75008 Paris (FR)
(72) Inventeur: LEDUC, Madeleine, F-75011 Paris (FR); RICHARD, Hervé, F-93420 Villepinte (FR); LAGRANGE, Alain, F-77700 Coupvray (FR)
(74) Mandataire: Dodin, Catherine
(86) Numéro de dépôt international: FR9900085
(87) Numéro de publication internationale: WO99037276

(56) Documents cités:
- PATENT ABSTRACTS OF JAPAN vol. 12, no. 11 (C-468) [2858], 13 janvier 1988 & JP 62 167771 A (MITSUBISHI PETROCHEM), 24 juillet 1987

## Description

La présente invention est relative à l'utilisation à titre d'agent protecteur des fibres kératiniques de polymères de polyammonium quaternaires hétérocycliques, à des compositions cosmétiques les mettant en oeuvre, ainsi qu'à des procédés de traitement des cheveux à l'aide de ces polymères.

On a déjà proposé d'utiliser des polymères comportant des groupements ammonium quaternaire comme agents de conditionnement pour la chevelure. De telles compositions sont décrites notamment dans les brevets français de la demanderesse n° 2 270 846, 2 307 271, et 2, 413 907.

Les polymères cationiques présentent une grande affinité pour les fibres kératiniques, tels que les cheveux, en raison de l'interaction des groupements cationiques avec les groupements anioniques du cheveu.

Ces polymères se déposent sur les cheveux d'autant plus facilement que ceux-ci sont plus sensibilisés et leur affinité pour les cheveux est souvent telle qu'ils résistent à l'élimination par les shampooings ou par brossage.

On a constaté, toutefois, que si l'utilisation de tels polymères cationiques présentait de nombreux avantages dans la mesure où ils facilitent le démêlage des cheveux et qu'ils leur confèrent des qualités de nervosité et un aspect brillant, en raison de leur affinité pour la kératine, ces polymères ont tendance à s'accumuler sur les cheveux à la suite d'applications répétées.

Les polymères cationiques à groupements quaternaires présentent, par ailleurs, souvent l'inconvénient d'être peu compatibles avec les agents tensio-actifs anioniques, ce qui réduit les possibilités d'utilisation et impose leur emploi dans des traitements à deux temps, avant ou après shampooing.

La demanderesse a découvert que certains polymères polyammonium quaternaires, qui ne présentent par les inconvénients mentionnés ci-dessus, comportant au moins des hétérocycles quaternaires insaturés étaient particulièrement intéressants pour le traitement des cheveux.

La demanderesse a notamment découvert que l'utilisation de ces polymères permettait de protéger les cheveux tant vis à vis des agressions, dûes notamment au soleil, aux intempéries, à la transpiration que vis à vis de celles résultant de traitement des cheveux tels que par exemple, les décolorations, permanentes ou teintures.

Il a été constaté que les fibres kératiniques avaient tendance à être fragilisées lorsqu'elles sont soumises à ces traitements; les cheveux deviennent secs, ternes et rugueux, difficiles à démêler et à coiffer.

Les agents protecteurs de l'invention sont utilisés notamment dans tout procédé cosmétique comportant au moins une étape au cours de laquelle les fibres kératiniques sont susceptibles d'être exposées à des agressions diverses et permettent ainsi d'éviter les inconvénients mentionnés ci-dessus.

Ces agents protecteurs peuvent être appliqués sur les fibres kératiniques, pendant, antérieurement ou postérieurement à cette étape au cours de laquelle les fibres kératiniques sont soumises à des agressions.

Préferentiellement, les agents protecteurs de la présente invention sont utilisés dans un procédé au cours duquel au moins une application d'une composition alcaline sur les fibres kératiniques a lieu.

L'invention a donc pour objet l'utilisation à titre d'agent protecteur de polymères polyquaternaires hétérocycliques.

L'invention a également pour objet des compositions cosmétiques les mettant en oeuvre et notamment des compositions destinées à une mise en forme permanente des fibres kératiniques, des compositions de décoloration et des compositions tinctoriales, de préférence d'oxydation.

L'invention a encore pour objet un procédé de traitement des cheveux mettant en oeuvre ces compositions.

D'autres objets de la présente demande ressortiront à la lecture de la description et des exemples qui suivent.

Les polymères utilisés à titre d'agent protecteur des fibres kératiniques sont constitués à base des motifs récurrents de formule (I): 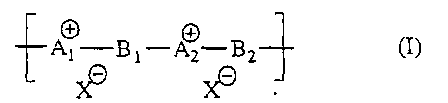 dans laquelle:
• A₁^{⊕}et A₂^{⊕}, identiques ou différents, désignent :
a) un hétérocycle insaturé quaternaire de formule (II): 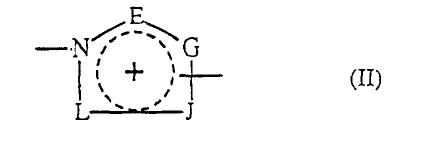 dans laquelle:
   E, G, L, J, identiques ou différents, désignent un atome de carbone, d'oxygène, de soufre ou d'azote, l'un au moins désignant un atome d'azote;
   E, G, L, J peuvent être substitués, lorsque un ou plusieurs de ces atomes désignent un atome de carbone, par ou plusieurs atomes d'halogène, groupements hydroxyle, nitro, cyano, sulfhydryle, carboxyle, un groupement alkyle, monohydroxyalkyle, polyhydroxyalkyle, thioalkyle, cyanoalkyle, alkoxy, acyle, acétyloxyl, un groupement cycloalkyle substitué ou non, aryle substitué ou non, alkylaryle substitué ou non ou de groupements -NHR_{N} dans lequel R_{N} désigne un groupement acétyle ou uréido;
   Lorsque E, G, L ou J désignent un troisième atome d'azote, celui-ci peut être substitué par un hydrogène, un radical alkyle, monohydroxy alkyle, polyhydroxy alkyle, aryle substitué ou non ou alkylaryle substitué ou non.
      Les substituants de deux des atomes E, G, L et J peuvent également former conjointement avec les atomes auxquels ils sont rattachés un cycle aromatique de 5 à 7 chaînons substitué ou non; ou
b) un ammonium quaternaire de formule (III) 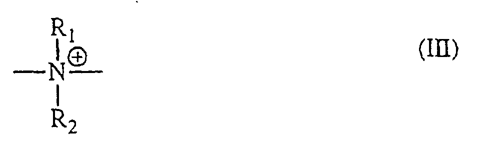 dans laquelle:
   R₁ et R₂, identiques ou différents, désignent un groupement carboxyle, un groupement alkyle, polyhydroxyalkyle, thioalkyle, cyanoalkyle, alkoxy, acyle, acétyloxy, un groupement cycloalkyle substitué ou non, aryle substitué ou non, alkylaryle substitué ou non ou un groupemement -NHR_{N} dans lequel R_{N} désigne un groupement acétyle ou uréido;
   R₁ et R₂ peuvent également former conjointement avec l'atome d'azote auquel ils sont rattachés un cycle saturé de 5 à 7 chaînons carbonés;
      et dans laquelle au moins un des groupements A₁^{⊕}ou A₂^{⊕}désigne un hétérocycle insaturé quaternaire de formule (II).

- B₁ et B₂, identiques ou différents, désignent un groupement hydrocarboné pouvant contenir, liés à ou intercalés dans la chaîne principale, un ou plusieurs cycles aromatiques substitué ou non, un ou plusieurs atomes d'oxygène, de soufre ou d'azote, un ou plusieurs groupements -SO- , -SO₂-, -SO₃H, amino, alkylamino, hydroxyle, ammonium quaternaire ou uréido.
- X^{⊖} représente un anion dérivé d'un acide organique ou minéral.

Les polymères de la présente invention ont un poids moléculaire moyen de préférence compris entre 1000 et 20000 en masse mesuré en chromatographie par perméation sur gel en prenant comme référence le poly éthylène glycol.

Dans le cadre de la présente invention:
Les atomes d'halogène désignent préférentiellement un atome de fluor, de chlore, de bromure ou d'iode.
Les radicaux alkyle, monohydroxyalkyle, polyhydroxyalkyle et les groupements hydrocarbonés peuvent être linéaires ou ramifiés.
Les groupements alkyle désignent notamment les groupements de 1 à 20 atomes de carbone, comme par exemple, les groupements méthyle, éthyle, propyle, isopropyle, n-propyle, butyle, n-butyle, tert-butyle, pentyle, n-pentyle, isopentyle, n-hexyle, isohexyle, heptyle, octyle, nonyle, decyle, undecyle, dodecyle et pentadecyle. Préférentiellement, les groupements alkyle désignent un groupement de 1 à 6 atomes de carbone;

Parmi les groupements hydrocarbonés, on peut citer les groupements polyméthylènes de 1 à 20 atomes de carbone.

Préférentiellement les groupements hydrocarbonés désignent les groupements polyméthylènes de 2 à 8 atomes de carbone.

Les groupements hydrocarbonés peuvent contenir liés à ou intercalés dans la chaîne principale un ou plusieurs cycles aromatiques, 1 ou plusieurs atomes d'oxygène, de soufre, d'azote, un ou plusieurs groupements -SO-, -SO₂-, -SO₃H, amino, alkylamino, hydroxyles, ammonium quaternaires ou uréido.

Parmi les groupements monohydroxyalkyle, on peut notamment citer les groupements hydroxyméthyle, hydroxyéthyle, hydroxypropyle et hydroxybutyle.

Parmi les radicaux polyhydroxyalkyle, on peut par exemple citer les radicaux dihydroxyéthyle, dihydroxypropyle, trihydroxypropyle et dihydroxybutyle.

Les radicaux thioalkyle désignent un groupement -R-SH, R représentant un groupement alkyle tel que défini ci-dessus.

Les radicaux cyanoalkyle désignent un groupement -R-C≡N, R représentant un groupement alkyl tel que défini ci-dessus.

Les groupements alcoxy désignent un groupement -O-R, R représentant un groupement alkyle tel que défini ci-dessus.

Les groupement acyle désignent un groupement - OC - R, R représentant un groupement alkyle tel que défini ci-dessus.

Les groupement acétyloxy désignent un groupement -O-CO-R, R représentant un groupement alkyle tel que défini ci-dessus.

Parmi les radicaux cycloalkyle, on peut notamment citer le cyclohexyle et le cyclopentyle.

Parmi les radicaux aryle on peut notamment citer les groupements phényle ou naphtyle.

Parmi les groupements alkylaryle, on peut notamment citer le groupement benzyle, phenethyle ou naphthylmethyle.

Parmi les cycles aromatiques, de 5 à 7 chaînons, on peut par exemple citer les cycles aryle, alkylaryle mentionnés ci-dessus. Les cycles aromatiques préférés sont les cycles phényle, pyrimidine, pyridine, pyrrole et pyrazole.

Dans le cadre de la présente invention, les radicaux cycloalkyles et les cycles aromatiques peuvent être substitués par un atome d'halogène, un groupement hydroxyle, amino, alkyl ou hydroxyalkyl en C₁-C₆.
- X^{⊖} représente notamment un anion dérivé d'un halogène tel que le chlore, le brome, le fluor ou l'iode, un anion dérivé d'acides minéraux tels que l'acide phosphorique ou l'acide sulfurique ou un anion dérivé d'un acide organique sulfonique ou carboxylique, notamment un acide alcanoïque ayant de 1 à 12 atomes de carbone tel que l'acide acétique, un acide phénylacanoïque tel que l'acide phénylacétique, l'acide benzoïque, l'acide citrique ou l'acide paratoluène sulfonique. Préférentiellement, X^{⊖} représente un anion dérivé d'un halogénure et plus préférentiellement X^{⊖} représente un anion chlorure ou bromure.

De préférence A₁ et/ou A₂ représente un hétérocycle de formule (II) comportant 2 atomes d'azote et 3 atomes de carbone.

Dans un mode de réalisation préféré de la présente invention, un au moins des groupements A₁^{⊕} ou A₂^{⊕} désigne un groupement imidazole quaternaire de formule (IV): 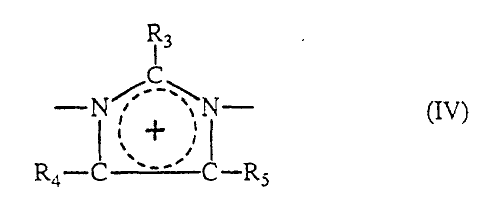 dans laquelle:
R₃, R₄ et R₅, identiques ou différents, désignent un atome d'hydrogène, d'halogène, un groupement hydroxyle, nitro, cyano, sulfhydryle, carboxyle, un groupement alkyle, monohydroxyalkyle, polyhydroxyalkyle, thioalkyle, cyanoalkyle, alkoxy, acyle, acétyloxyl, un groupement cycloalkyle, aryle, alkylaryle ou un groupement -NHR_{N} dans lequel R_{N} désigne un groupement acétyle ou uréido; les radicaux R₄ et R₅ peuvent également former conjointement avec les atomes auxquels ils sont rattachés un cycle aromatique de 5 à 7 chaînons.

Dans une autre forme de réalisation de la présente invention, au moins un des groupements A₁^{⊕} ou A₂^{⊕} désigne un groupement pyrazole quaternaire de formule (V): 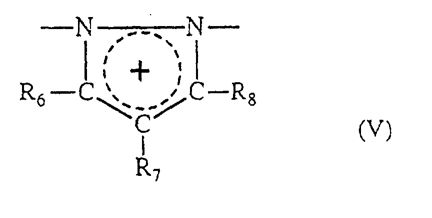 dans laquelle :
R₆, R₇ et R₈, identiques ou différents, désignent un atome d'hydrogène, d'halogène, un groupement hydroxyle, nitro, cyano, sulfhydryle, carboxyle, un groupement alkyle, monohydroxyalkyle, polyhydroxyalkyle, thioalkyle, cyanoalkyle, alkoxy, acyle, acétyloxyl, un groupement cycloalkyle, aryle, alkylaryle ou un groupement -NHR_{N} dans lequel R_{N} désigne un groupement acétyle ou uréido;
deux des radicaux R₆, R₇ ou R₈ peuvent également former conjointement avec les atomes auxquels ils sont rattachés un cycle aromatique de 5 à 7 chaînons.

Dans un autre mode de réalisation préféré de la présente invention, les groupements A₁^{⊕} et A₂^{⊕} désignent simultanément un groupement imidazole quaternaire de formule (IV), dans laquelle R₃, R₄ et R₅, identiques ou différents désignent un atome d'hydrogène, un groupement hydroxyle, nitro, cyano, sulfhydryle, carboxyle, un groupement alkyle, monohydroxyalkyle ou polyhydroxyalkyle; les radicaux R₄ et R₅ peuvent également former conjointement avec les atomes auxquels ils sont rattachés un cycle aromatique de 5 à 7 chaînons;

Dans une autre forme particulièrement préférée de la présente invention, les groupements A₁^{⊕} et A₂^{⊕} désignent simultanément un groupement imidazole quaternaire de formule (IV) dans laquelle R₃, R₄ et R₅, identiques ou différents, désignent un atome d'hydrogène, un groupement alkyle en C₁ à C₆ et les radicaux R₄ et R₅ peuvent former conjointement avec les atomes auxquels ils sont rattachés un cycle phényle.

Dans un autre mode de réalisation préféré de la présente invention, A₁^{⊕} représente un ammonium quaternaire de formule (III) dans laquelle R₁ et R₂, identiques ou différents, désignent un atome d'hydrogène ou un groupement alkyle en C₁ à C₆ ou forment conjointement avec l'azote un groupement à six chaînons et A₂^{⊕} désigne un groupement imidazole quaternaire de formule (IV) dans laquelle R₃, R₄ et R₅, identiques ou différents désignent un atome d'hydrogène, un alkyle en C₁-C₆.

Dans une forme de réalisation préférée de l'invention, au moins un des groupements B₁ ou B₂ désigne un groupement de formule (VI)

―(CH₂)ₙ―CO―A―OC―(CH₂)ₙ― (VI)

dans laquelle :
n désigne un nombre entier de 1 à 10, et préférentiellement un nombre entier de 1 à 6,
A désigne :
   (a) un reste de glycol de formule: -O-Z-O-
      dans laquelle Z désigne un groupement hydrocarboné ou un groupement répondant aux formules: 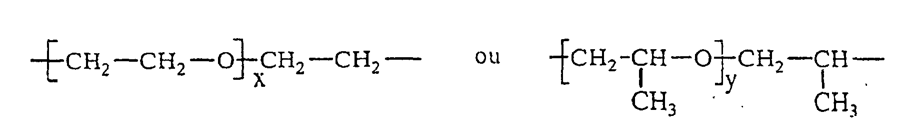 dans lesquelles x et y désignent un nombre quelconque de 1 à 4; ou
   (b) un reste de diamine bis-secondaire tel qu'un dérivé de pipérazine; ou
   (c) un reste de diamine bis-primaire de formule :

      ―NH―Y―NH―

      dans laquelle Y désigne un groupement hydrocarboné ou un radical

      -(CH₂)₂―S―S―(CH₂)₂― ;

      ou
   (d) un groupement uréylène, c'est à dire un groupement de formule -NH-CO-NH- .

Dans un mode de réalisation préféré, B₁ et B₂, identiques ou différents, désignent un groupement polyméthylène pouvant contenir un ou plusieurs atomes d'oxygène ou un ou plusieurs cycles aromatiques.

Dans un mode de réalisation particulièrement préféré, B₁ et B₂, identiques ou différents désignent un groupement de formule :

-(CH₂)ₙ - ;

n désignant un nombre entier de 1 à 6, ou 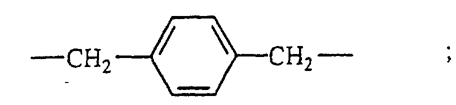 ou

―(CH₂)₂―O―(CH₂)₂―O―(CH₂)₂―

Préférentiellement, dans les polymères mentionnés ci-dessus, X^{⊖} représente un halogénure.

Certains polymères utilisés ci-dessus sont nouveaux en soit et constituent à ce titre un autre objet de l'invention.

Une famille de ces polymères nouveaux est constitué par les polymères à base des motifs récurrents de formule (VII) : 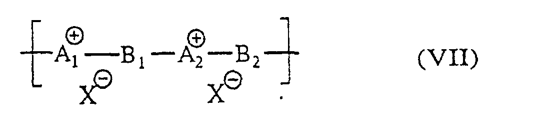 dans laquelle :
A₁^{⊕} désigne un hétérocycle insaturé quaternaire de formule (II) telle que définie ci-dessus et A₂^{⊕} désigne un ammonium quaternaire de formule (III); B₁, B₂, X^{⊖} et les formules (II) et (III) étant tels que définis ci-dessus et au moins un des groupements B₁ ou B₂ est différent de - CH₂ - CHOH - CH₂-.

Dans la formule (VII) les hétérocycles insaturés quaternaires de formule (II), les groupements imidazole quaternaires de formule (IV) telle que définie ci-dessus et les groupements pyrazole quaternaire de formule (V) telle que définie ci-dessus sont préférés.

Une famille de polymères nouveaux est constituée à base des motifs récurrents de formule (VIII): 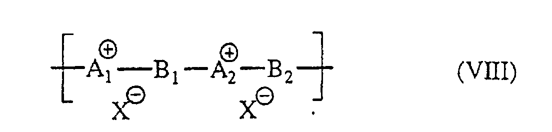 dans laquelle
A₁^{⊕} désigne un groupement imidazole quaternaire de formule (IV) tel que défini ci-dessus et A₂^{⊕} désigne un groupement pyrazole quaternaire de formule (V) tel que défini ci-dessus; B₁, B₂ et X^{⊖} étant tels que définis ci-dessus.

Une autre famille de polymères nouveaux est représentée par les polymères constitués à base de motifs récurrents de formule (IX) : 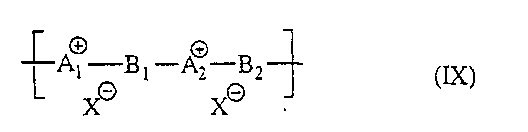 dans laquelle A₁^{⊕} est tel que défini dans la formule (I) et dans laquelle A₂^{⊕} désigne un groupement imidazole quaternaire de formule (IV) dans laquelle R₄ et R₅, identiques ou différents sont tels que définis dans la formule (IV) mentionnée ci-dessus et R₃ désigne un groupement cycloalkyle, aryle ou alkylaryle et préférentiellement un groupement phényle; B₁, B₂, X^{⊖} étant définis ci-dessus.

Parmi les composés nouveaux (IX), ceux pour lesquels A₁^{⊕} désigne un imidazole quaternaire de formule (IV), et plus particulièrement lorsque le groupement R₃ de (IV) désigne un phényle, sont particulièrement préférés.

Parmi les composés nouveaux préférés (VII), (VIII) et (IX), B₁ et/ou B₂ désigne préférentiellement un groupement de formule (VI):

―(CH₂)ₙ―CO―A―OC―(CH₂)ₙ― (VI)

dans laquelle:
n désigne un nombre entier de 1 à 10;
A désigne :
   (a) un reste de glycol de formule: -O-Z-O-
      dans laquelle Z désigne un groupement hydrocarboné, ou un groupement répondant aux formules: 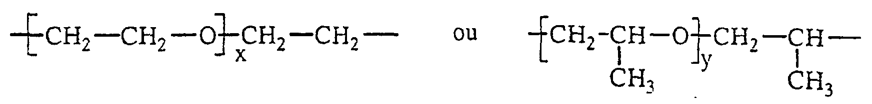 dans lesquelles x et y désignent un nombre quelconque de 1 à 4; ou
   (b) un reste de diamine bis-secondaire; ou
   (c) un reste de diamine bis-primaire de formule :

      ―NH―Y―NH―

      dans laquelle Y désigne un groupement hydrocarboné ou le radical

      ―(CH₂)₂―S―S―(CH₂)₂ ― ;

      ou
   (d) un groupement uréylène
et plus particulièrement -(CH₂)ₙ - , n désignant un nombre entier de 1 à 6; ou 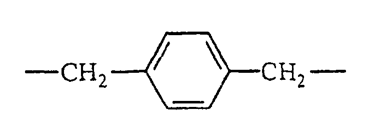 ou

―(CH₂)₂―O―(CH₂)₂―O―(CH₂)₂―

et au moins un des groupements B₁ ou B₂ étant différent de -CH₂-CROH-CH₂-, avec R = H ou alkyle et les groupements B₁ et B₂ étant différents du groupement:

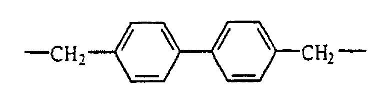

La synthèse des composés de la présente invention est réalisée en deux étapes. La première étape consiste à synthétiser la diamine puis la deuxième étape consiste en la quaternisation de la diamine en présence d'un dihalogénure ou d'un disulfonate.

La synthèse de la diamine est réalisée par la réaction du diazole correspondant avec un dihalogénure ou un disulfonate dans un solvant, en présence d'une base, à une température comprise entre la température ambiante et le reflux.

Ces solvants peuvent être l'eau, des aromatiques comme le benzène, le toluène, le diméthylsulfoxyde, le tétrahydrofurane, le diméthylformamide.

Ces solvants peuvent aussi être utilisés en mélange.

Les bases peuvent consister en des hydroxydes tels que la soude, la potasse ou en des amidures, des carbonates ou des hydrures.

Cette synthèse peut aussi avoir lieu en transfert de phase par l'ajout d'un agent de transfert de phase.

Des synthèses sont décrites notamment dans les documents J. Elguero and All, Journal of Hétérocyclic Chemistry 25, 771-782 (1988), Yin-hung So, Macromolécules 25,516-520 (1992) et R.G. Xie and all, Chinese Chemical Letters 7, 321-324 (1996).

La quaternisation avec un dihalogénure ou un disulfonate est réalisée dans un solvant à une température comprise entre la température ambiante et le reflux. Les solvants peuvent être choisis parmi l'eau, des alcools, des aromatiques comme le benzène, le toluène, le diméthylsulfoxyde, le tétrahydrofurane ou le diméthylformamide. Ces solvants peuvent également être utilisés en mélange. Préférentiellement, ils sont choisis parmi l'eau, les alcools et les mélanges hydroalcooliques.

Les polymères hétérocycliques insaturés quaternaires utilisés conformément à l'invention pour la protection des cheveux sont utilisés notamment dans des compositions utilisées pour la déformation permanente des cheveux, de coloration ou de décoloration qui constituent un autre objet de l'invention.

La technique la plus usuelle pour obtenir une déformation permanente des cheveux consiste dans un premier temps à appliquer une composition contenant un agent réducteur sur les fibres kératiniques, puis dans un deuxième temps, à appliquer sur les cheveux préalablement mis sous tension par des bigoudis ou d'autres moyens, une composition oxydante de façon à donner finalement aux cheveux la forme recherchée.

Les agents protecteurs de la présente invention peuvent être contenus dans la composition réductrice et/ou oxydante.

Les compositions réducrices conformes à l'invention comprennent dans un milieu approprié pour la déformation permanente des cheveux au moins un agent réducteur susceptible de rompre les liaisons disulfures (- S - S -) au niveau des cheveux et au moins un polymère hétérocyclique insaturé quaternaire tel que défini ci-dessus.

Les agents réducteurs sont choisis généralement parmi les sulfites, les bisulfites ou les thiols.

Parmi les agents réducteurs préférés, on peut citer la cystéine, la cystéamine et leurs dérivés tels que leurs sels cosmétiquement acceptables comme les chlorhydrates, bromhydrates, citrates, acétates, sulfates, l'acide thiolactique, l'acide thioglycolique, ainsi que leurs esters, notamment le thioglycolate de glycérol.

Les agents réducteurs sont présents dans des proportions suffisantes pour réduire les liaisons disulfures de la kératine, de préférence comprises entre 1 et 25%, et en particulier entre 1 et 10% en poids.

Les agents protecteurs de la présente invention peuvent être présents dans des proportions comprises entre 0,01% et 10% en poids de la composition réductrice, et préférentiellement entre 1% et 5%.

Le pH des compositions réductrices est ajusté de façon à obtenir un pH compris entre 6,5 et 11,5.

Les agents alcalins sont choisis de préférence parmi la monoéthanolamine, la diéthanolamine, la triéthanolamine, l'isopropylamine, la 2méthyl-amino-propanol-1, la propane diamine 1,3, un carbonate ou un bicarbonate alcalin ou d'amminium, l'ammoniaque, un carbonate organique tel que le carbonate de guanidine ou encore un hydroxyde alcalin, utilisé seul ou en mélange.

Cette composition réductrice peut également contenir des agents tensioactifs non-ioniques, anioniques, cationiques ou amphotères, couramment utilisés dans de telles compositions. Parmi ceux-ci ont peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alkylpolyglucosides, les alcanolamides d'acides gras, les esters d'acides gras oxyéthylénés, ainsi que des tensio-actifs non-ioniques de la famille des hydroxypropyléthers.

Ces agents tensio-actifs sont généralement utilisés dans des proportions maximales de 30%, et de préférence comprises entre 0,5 et 10% en poids par rapport au poids total de la composition.

Ces compositions peuvent aussi contenir des agents épaississants tels que la gomme de guar, la gomme de Tera, la farine d'épicéa.

Ces compositions peuvent également contenir des agents traitants tels que des silicones volatiles ou non, linéaires ou cycliques ou leurs mélanges. Parmi les silicones, on peut citer les polydiméthyl-siloxanes, les polyorganosiloxanes quaternisés tels que décrits dans FR-A-2 535 730, les polyorganosiloxanes à groupement aminoalkyle modifiés par des groupements alcoxycarbonylalkyle tels que décrits dans le brevet US-A-4 749 732, les polyorganosiloxanes tels que les copolymères de polydiméthylsiloxane-polyoxyalkyle tels que le diméthicone copolyol, un polydiméthylsiloxane à groupements terminaux stéaroxy-(stéaroxydiméthicone), un copolymère polydiméthylsiloxane dialkylammonium acétate ou un copolymère polydiméthylsiloxane polyalkylbétaïnc décrit dans GB-A-2 197 352, des polysiloxanes organomodifiés par des groupements mercapto ou mercapto-alkyle tels que décrits dans FR-B-1 530 369 et EP-A-0 295 780, ainsi que des silanes tels que le. stéaroxy-triméthylsilane.

D'autres ingrédients utilisables dans les compositions réductrices contenant les agents protecteurs de l'invention sont choisis parmi les cires, les polymères choisis parmi les polymères cosmétiquement acceptables anioniques, cationiques autres que ceux de l'invention, non-ioniques ou amphotères, les agents de gonflement et de pénétration permettant de renforcer l'efficacité du réducteur tels que le diméthylisosorbitol, l'urée et ses dérivés, la pyrrolidone, les n-alkylpyrrolidone, la thiamorpholinone, les alkyléthers d'alkylèneglycol ou de dialkylèneglycol tels que par exemple le monométhyléther de propylèneglycol, le monométhyléther de dipropylèneglycol, des alcanediols en C₃-C₆ tels que le propanediol-1,2, l'imidazolidinone-2, ainsi que d'autres composés tels que des alcools gras, des dérivés de lanoline, des céramides, notamment les céramides elles-mêmes, les glycocéramides, les pseudocéramides décrits notamment dans FR-A-95 12399, et dans DOWNING Journal of Lipid Research, Vol. 35, p. 2060, 1994, ou dans FR-A-2 673 179, EP-A-0227994, WO-94/07844, WO-92/05764, des ingrédients actifs tels que l'acide pantothénique, le panthénol, des agents anti-chute, des agents anti-pelliculaires, des agents de mise en suspension, des agents séquestrants, des agents opacifiants, des colorants, des filtres solaires siliconés ou non, ainsi que des parfums et des conservateurs.

Les agents protecteurs définis ci-dessus peuvent également être présents dans des compositions oxydantes utilisées lors de la mise en forme permanente des fibres kératiniques. L'invention a donc aussi pour objet une composition oxydante pour la mise en forme permanente des fibres kératiniques comprenant dans un milieu approprié pour la permanente, un agent oxydant et un polymère tel que défini ci-dessus.

Les agents oxydants peuvent être choisis parmi l'eau oxygénée, les peroxydes d'urée, les bromates tels que les bromates alcalins, les persels ou un mélange de bromates alcalins et d'un persel.

Lorsque l'agent oxydant est constitué par l'eau oxygénée, il est présent dans des proportions comprises entre 1 et 10 volumes, et de préférence de l'ordre de 8 volumes.

Lorsque les bromates sont utilisés, la concentration en bromates alcalins est de 1 à 12% et celle en persels de 0,1 à 15% en poids par rapport au poids total de la composition oxydante.

Les agents protecteurs de la présente invention peuvent être présents dans des proportions comprises entre 0,01% et 10% en poids de la composition oxydante, et prérérentiellement entre 1% et 5% en poids.

Le pH de ces compositions est compris habituellement entre 2 et 9, et de préférence entre 3 et 8; il est de préférence acide.

Lorsque l'eau oxygénée est utilisée, elle peut être stabilisée par la phénacétine, l'acétaniline, les phosphates mono- et trisodiques ou par les sulfates d'hydroxy-8 quinoléine.

Un autre objet de l'invention est un procédé de mise en forme permanente des fibres kératiniques et en particulier des cheveux, essentiellement caractérisé par le fait :
- qu'on applique sur les fibres kératiniques, de préférence mouillées, une composition réductrice de la kératine, la composition réductrice étant appliquée sur les fibres mises en forme,
- qu'après un temps de pose suffisant pour réduire la kératine, on applique une composition oxydante,
- qu'on procède au rinçage, de préférence à l'eau, après un temps de pose suffisant pour fixer les fibres mises en forme et réduites lors de la première étape sous une forme permanente; la composition réductrice et/ou la composition oxydante étant telles que définies ci-dessus.

Les cheveux sont mis en forme par des moyens divers tels que rouleaux, pinces, bandes à crochets, ou simplement à la main.

Un autre objet de la présente invention est une composition pour la teinture directe des fibres kératiniques et en particulier des fibres kératiniques humaines telles que les cheveux, comprenant dans un milieu approprié pour la teinture, au moins un polymère tel que défini ci-dessus, et au moins un colorant direct.

Parmi les colorants directs classiquement utilisés, on peut citer des colorants nitrés benzéniques, tels que les nitrophénylènediamines, les nitrodiphénylamines, les nitroanilines, les éthersde phénol nitrés ou les nitrophénols, des nitropyridines, des colorants anthraquinoniques, mono- ou di-azoïques, triarylméthaniques, aziniques, acridiniques et xanthéniques ou encore des colorants métallifères.

Les colorants directs plus particulièrement préférés selon l'invention sont choisis parmi les suivants:
i) les colorants nitrés benzéniques de formule (A) suivante: 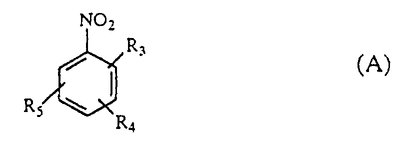 dans laquelle
   - R₃ désigne un radical NH₂, amino monosubstitué par un radical alkyle, monohydroxyalkyle, polyhydroxyalkyle, aminoalkyle, ou amino disubstitué par des radicaux, identiques ou différents, alkyle, mono- ou poly-hydroxyalkyle, ou aminoalkyle,
   - R₄ désigne hydrogène, hydroxy, alcoxy, mono- ou polyhydroxyalkyloxy, ou les mêmes significations désignées ci-dessus pour R₃, à l'exception du radical amino disubstitué,
   - R₅ désigne hydrogène, alkyle, nitro, ou halogène,
ii) les colorants anthraquinoniques de formule (B) suivante: 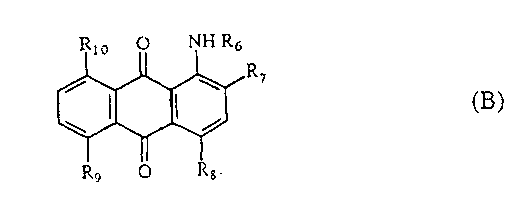 dans laquelle
   - R₆ désigne hydrogène, un radical monohydroxyalkyle ou polyhydroxyalkyle,
   - R₇ désigne hydrogène, un radical alkyle ou alcoxy,
   - R₈ désigne hydrogène, un radical hydroxy, amino ou monohydroxyalkylamino ou polyhydroxyalkylamino,
   - R₉ et R₁₀, identiques ou différents sont hydrogène, hydroxy ou amino,
iii) les colorants azoïques de formule (C) suivante: 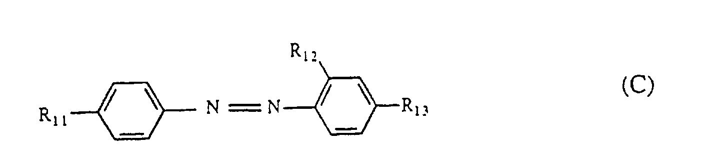 dans laquelle:
   - R₁₁ désigne un radical nitro, amino, amino mono- ou di-substitué par des alkyles,
   - R₁₂ désigne hydroène ou un radical alkyle,
   - R₁₃ désigne un radical amino, amino mono- ou di-substitué par des monohydroxyalkyles,
      étant entendu que les radicaux alkyles et alcoxy cités ci-avant dans les formules (A), (B) et (C) sont en C₁-C₄ et qu'ils peuvent être linéaires ou ramifiés, et les sels cosmétiquement acceptables de tous ces composés.

Par C₁-C₄, on entend notamment les radicaux méthyle, éthyle, n-propyle, isopropyle, n-butyle, sec-butyle, et tert-butyle Par sels cosmétiquement acceptables, on désigne plus particulièrement les chlorhydrates, bromhydrates, et les sulfates.

Plus avantageusement encore, selon la présente invention, on préfère mettre en oeuvre les colorants directs suivants:
- 1-amino-2-nitro-4-N-(β-hydroxyéthyl)-amino-5-méthyl-benzène,
- 1,4,5,8-tétraaminoanthraquinone,
- 1,4-bis-N,N'-[(β,γ-dihydroxypropyl)-amino]-anthraquinone,
- 1,4,4-N-tris(β-hydroxyéthyl)-1,4-diamino-2-nitro-benzène,
- 1-N-(β-hydroxyéthyl)-amino-2-nitro-4-amino-benzène,
- 1-hydroxy-3-nitro-4-amino-benzène
- 1-hydroxy-3-nitro-4-N-(β-hydroxyéthyl)-amino-benzène,
- 1-(β-hydroxyéthyloxy-3-méthylamino-4-nitro-benzène,
- 1-méthylamino-2-nitro-5-β,γ-dihydroxypropyloxy-benzène,
- 1-N-(β-aminoéthyl-amino-2-nitro-4-β-hydroxyéthyloxy-benzène,
- 4-[N-éthyl-N-(β-hydroxyéthyl)-amino]-1-N-(β-hydroxyéthyl)-amino-2-nitro-benzène
- 1-4'-amino-diphénylazo)-2-méthyl-4-N-bis-(β-hydroxyéthyl-amino-benzène,
- 1-méthoxy-3-N-(β-aminoéthyl)-amino-4-nitro-benzène,
- 1-amino-2-nitro-4-N-(β-hydroxyéthyl)-amino-benzène,
- 1-amino-2-nitro-4-N-bis-(β-hydroxyéthyl)-amino-benzène,
- 1, 4-N-bis(β-hydroxyéthyl)-amino-5-nitro-benzène,
- 1,4-diamino-anthraquinone,
et leurs sels cosmétiquement acceptables

D'autres colorants cationiques préférés sont ceux de type ARIANOR (BASIC BROWN 17, BASIC BROWN 16, BASIC YELLOW 57, BASIC BLUE 99) ainsi que les colorants canoniques décrits dans les brevets CIBA WO 95/01772, WO 95/15144 et EP 714 954.

Les colorants directs, sous forme de base ou salifiée, sont généralement présents dans la composition tinctoriale selon l'invention dans des proportions pouvant aller d'environ 0,001 à environ 10%, et de préférence d'environ 0,05 à environ 5% en poids par rapport au poids total de la composition.

La présente invention est aussi relative à une composition pour la teinture d'oxydation des fibres kératiniques et en particulier des fibres kératiniques humaines telles que les cheveux, contenant dans un milieu approprié pour la teinture, au moins un polymère tel que défini ci-dessus et au moins un précurseur de colorant d'oxydation et/ou des précurseurs de la mélanine.

Les précurseurs de colorants d'oxydation peuvent notamment être choisis parmi les paraphénylènediamines, les para-aminophénols, les orthophénylènediamines et les bases hétérocycliques telles que par exemple les dérivés pyridiniques, les dérivés pyrimidiniques, les dérivés pyrazoliques, les dérivés pyrazolo-pyrimidiniques, des indoles ou des indolines et leurs sels d'addition d'acides.

Ces compositions peuvent aussi renfermer des coupleurs qui peuvent notamment être choisis parmi les méta-phénylènediamines, les méta-aminophénols, les métadiphénols, les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

D'une manière générale, les sels d'addition avec un acide utilisables dans le cadre des compositions tinctoriales sont notamment choisis parmi les chlorhydrates, les bromhydrates, les sulfates, les citrates, les succinates, les tartrates, les lactates et les acétates.

Le milieu approprié pour la teinture directe ou d'oxydation (ou support) est généralement constitué par de l'eau ou par un mélange d'eau et d'au moins un solvant organique pour solubiliser les composés qui ne seraient pas suffisamment solubles dans l'eau.

A titre de solvant organique, on peut par exemple citer les alcanols inférieurs tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Lorsqu'elles sont présentes, la ou les bases d'oxydation représentent de préférence de 0,0005 à 12 % en poids environ du poids total de la composition tinctoriale.

Lorsqu'ils sont présents, le ou les coupleurs représentent de préférence de 0,0001 à 10 % en poids environ du poids total de la composition tinctoriale.

Les polymères de l'invention représentent de préférence de 0,01 à 10% en poids environ du poids total de la composition tinctoriale directe ou oxydante, et préférentiellement de 0,1 à 5%.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH des compositions tinctoriales est généralement compris entre 3 et 12 environ, et de préférence entre 5 et 11. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 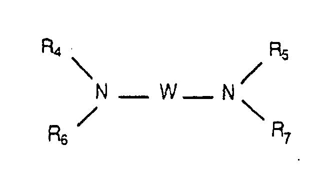 dans laquelle W est un reste propylène éventuellement substitué par un groupement hydroxyle ou un radical alkyle en C₁-C₆ ; R₄, R₅, R₆ et R₇, identiques ou différents, représentent un atome d'hydrogène, un radical alkyle en C₁-C₆ ou hydroxyalkyle en C₁-C₆.

Les compositions tinctoriales comprenant un agent protecteur peuvent également renfermer divers adjuvants utilisés classiquement dans les compositions pour la teinture des cheveux.

Les compositions d'oxydation utilisées dans la coloration par oxydation qui constituent un autre objet de l'invention peuvent également contenir un polymère hétérocyclique insaluré quaternaire tel que défini ci-dessus et au moins un agent oxydant.

Ces agents oxydants peuvent notamment être choisis parmi le peroxyde d'hydrogène, le peroxyde d'urée, les bromates de métaux alcalins, les persels tels que les perborates et persulfates, et les enzymes telles que les peroxydases et les oxydo-réductases à deux électrons.

L'invention a également pour objet un procédé de teinture des cheveux, caractérisé par le fait que l'on applique sur les cheveux en une quantité suffisante pour teindre les cheveux, une composition telle que définie ci-dessus, contenant l'agent protecteur conforme à l'invention éventuellement avec une composition oxydante, dans le cas de la teinture d'oxydation. Dans ce dernier cas, seule la composition contenant les précurseurs de colorant ou la composition oxydante peut renfermer un polymère selon l'invention. On laisse agir la composition pendant un temps compris entre 5 et 45 minutes environ puis on rince les cheveux.

Pour la teinture directe, on applique sur les cheveux dans une quantité suffisante pour produire une coloration une composition contenant au moins un colorant direct telle que définie ci-dessus que l'on laisse agir pendant une durée comprise entre 10 et 60 mn environ. On procède ensuite à un rinçage des cheveux.
Il est aussi possible d'effectuer des colorations directes sans rinçage.

La présente invention a aussi pour objet une composition de décoloration contenant, dans un milieu approprié pour la décoloration un agent décolorant les cheveux et un agent protecteur tel que défini ci-dessus.

Pour la décoloration des cheveux, on utilise les agents décolorants connus en eux-mêmes, tels que le peroxyde d'hydrogène, les persulfates, les percarbonates de sodium, les perborates.

Pour décolorer les cheveux, on applique sur les cheveux une composition décolorante telle que définie ci-dessus dans une quantité et pendant une durée suffisante pour décolorer les cheveux. Les cheveux sont ensuite rincés.

L'invention concerne également des compositions destinées au traitement cosmétique des cheveux contenant un polymère hétérocyclique quaternaire nouveau tel que défini dans les formules VII, VIII et IX.

Les exemples qui suivent sont destinés à illustrer l'invention:

Les tableaux 1 et 2 ci-dessous sont destinés à illustrer la préparation de polymères utilisés selon l'invention.

Ces tableaux reprennent successivement les indications relatives à la structure du composé du polymère de formule (I).

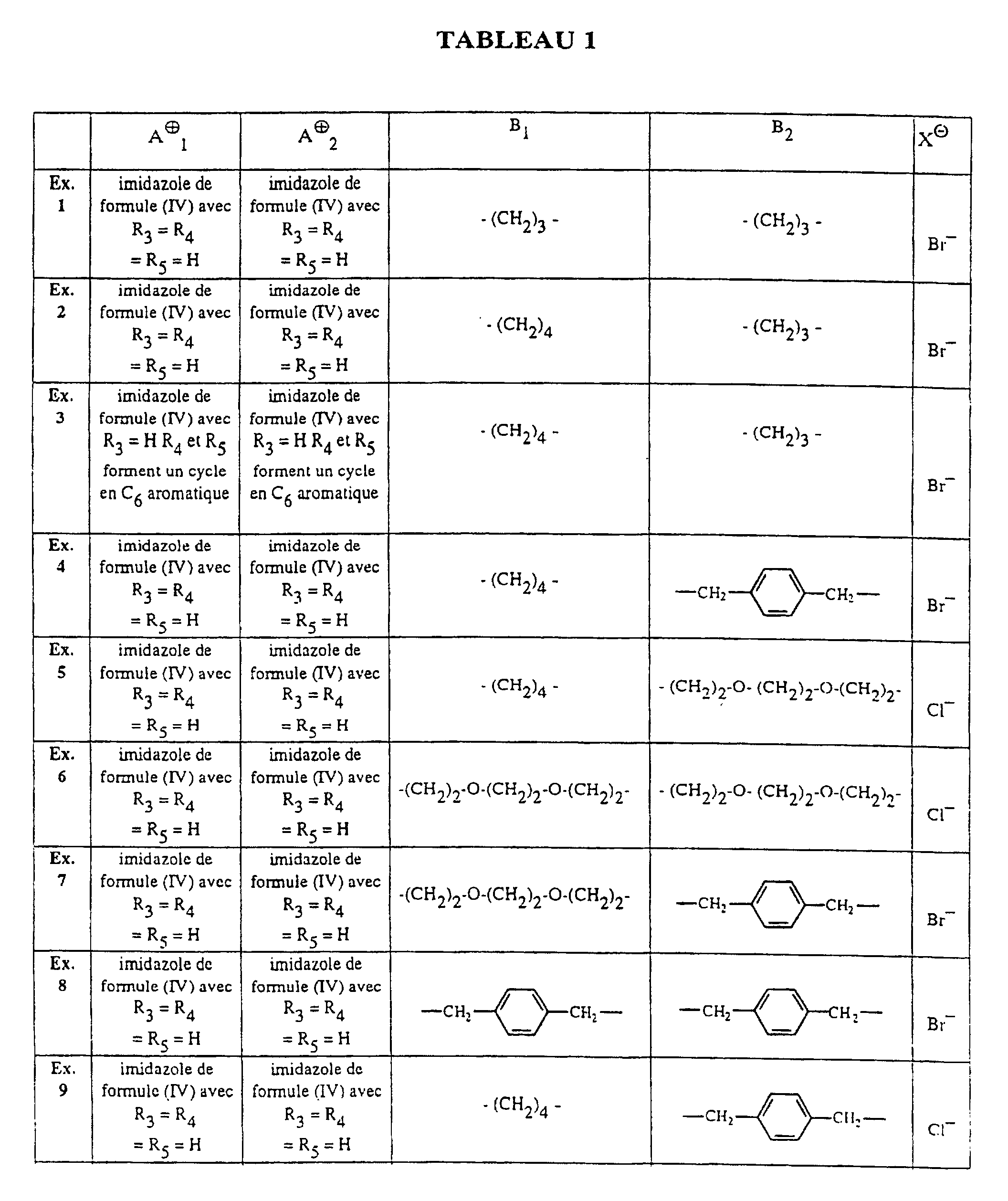

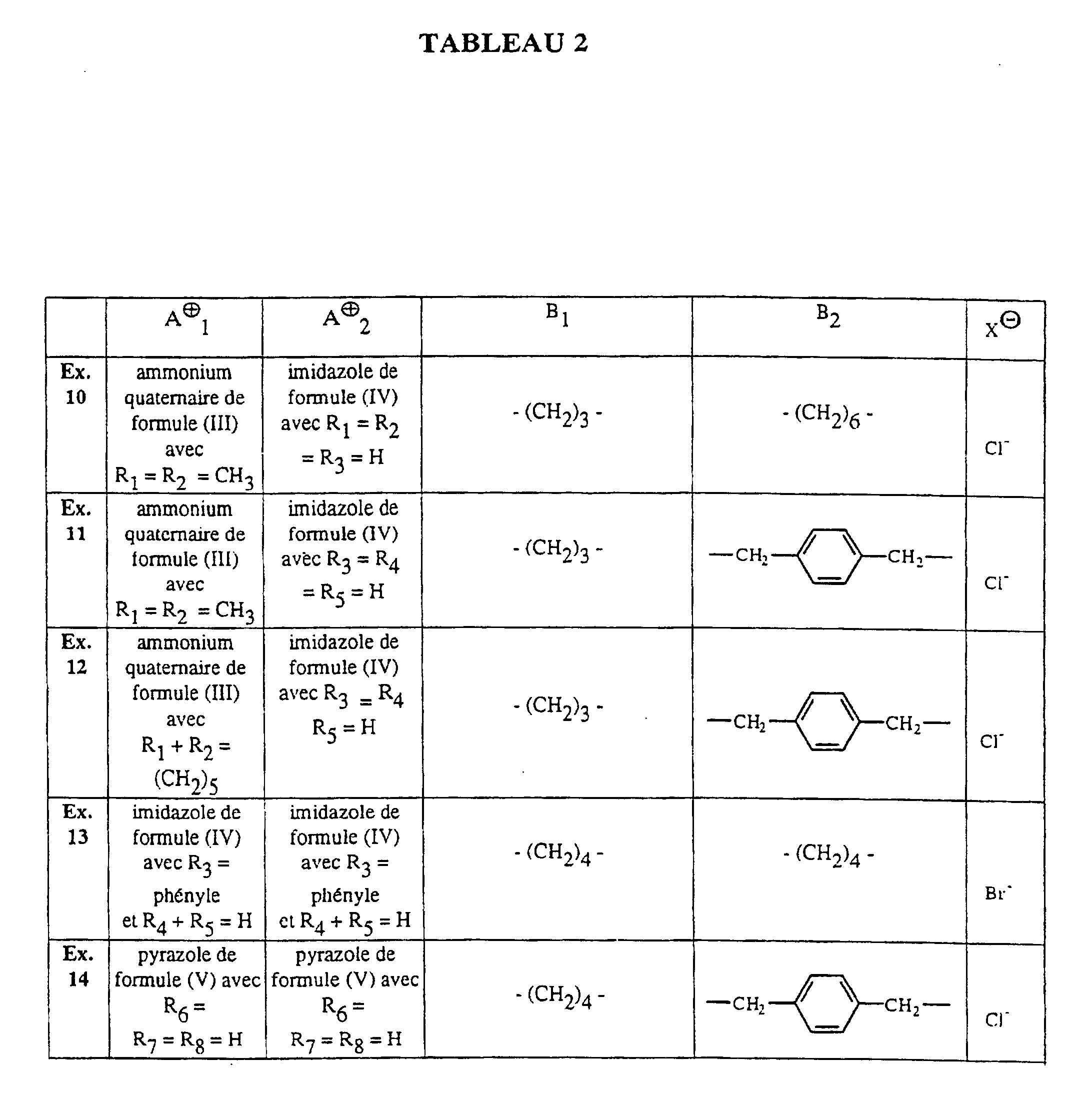

### 1) Synthèse de l'exemple 1

### a) Synthèse de la diamine 1,1'-(1,3-Propanediyl)bis(imidazole)

Dans un réacteur équipé d'une agitation mécanique, d'un thermomètre et d'un réfrigérant, on introduit 35,4g (0.52 mole) d'imidazole, une solution de 100 g (2.5 moles) de soude dans 100 ml d'eau, 5.15 g de bromure de tétrabutylammonium et 52.49 g (0.26 mole) de dibromo 1,3 propane en solution dans 240 ml de toluène. Ce mélange est porté au reflux pendant 32 heures. Après refroidissement, on ajoute au mélange 150 ml d'eau. Le mélange réactionnel présente alors trois phases liquides. Après décantation, on récupère la phase intermédiaire, insoluble dans les solvants organiques usuels, et on la distille. 27.5 g d'huile transparente, légèrement jaune sont ainsi séparés.
Les résultats obtenus par RMN proton DMSO sont les suivants δ 2.22 (m,2H); δ 3,92(t,4H); δ 6,91 (m,2H); δ 7.17 (m,2H);δ 7.61 (m,2H)

### b) Synthèse du polymère :

On porte au reflux pendant 10 heures un mélange de 17.6 g (0,1 mole) de diamine obtenue ci-dessus, 100 ml de méthanol et 20,2 g (0,1 mole) de dibromo-1,3 propane. Après évaporation du solvant, le résidu est broyé dans 300 ml d'éther isopropylique jusqu'à l'obtention d'une poudre qui est filtrée et séchée sous vide en présence d'anhydride phosphorique. On obtient ainsi 34,6g d'une poudre beige hydroscopique avec un rendement de 91,5%.
Dosage des bromures par AgNO3: 40.4 % (théorie : 42,3 %)

### 2) Synthèse de l'exemple 2 :

### a) Synthèse de la diamine 1.1'-(1,4-Butanediyl)bis(imidazole)

Dans un réacteur équipé d'une agitation mécanique, d'un thermomètre, d'un réfrigérant, on introduit 35,4 g (0,52 mole) d'imidazole, une solution de 100 g (2,5 moles) de soude dans 100 ml d'eau, 5.15 g de bromure de tétrabutylammonium, et 54g (0,26 mole) de dibromo 1,4 butane en solution dans 240 ml de toluène. Ce mélange est porté au reflux pendant 6 heures. Après refroidissement, la diamine attendue critallise, on la filtre, on lave le gâteau obtenu avec 100 ml de toluène, puis avec 2 fois 50 ml d'eau glacée. On recristallise le produit ainsi lavé dans 200 ml d'eau. Après séchage on obtient 44 g de poudre beige clair (rendement: 89%).
Une analyse par RMN proton DMSO a donné les résultats suivants :
δ 1,61-1,68 (m,4H); δ 3,96-4.02 (m,4H); δ 6,91 (m,2H);
δ 7,17 (m,2H); 7,64 δ (m,2H)

### b) Synthèse du polymère

On porte au reflux pendant 56 heures un mélange composé de 24,73 g (0,13 mole) de diamine obtenue ci-dessus, 210 ml de méhanol et 26,25 g (0,13 mole) de dibromo-1,3 propane. Après évaporation du solvant, le résidu est broyé dans 300 ml d'éther isopropylique jusqu'à l'obtention d'une poudre qui est filtrée et séchée sous vide en présence d'anhydride phosphorique. La poudre beige obtenue (39,9 g; rendement: 100%) est hygroscopique.
Dosage des bromures par AgNO3 : 38% (théorie : 40,7%).

### 3) Synthèse de l'exemple 3 :

### a) Synthèse de la diamine 1,1'-(1,4-Butanediyl)bis(benzimidazole)

A un mélange de 31,4 g (0,78 mole) de potasse broyée de 14 g (0,12 mole) de benzimidazole et de 200 ml d'acétone, on ajoute goutte à goutte, 12,95 g (0,06 mole) de dibromo 1,4-butane à température ambiante et on maintient l'agitation pendant 8 heures à cette température; on filtre alors le précipité formé et on le recristallise directement dans 50 ml d'éthanol. On obtient ainsi 9,7 g de poudre blanche avec un point de fusion à 175°C.
L'analyse RMN proton CDC13 a donné les résultats suivants :
δ 1,87-1,92 (m,4H); δ 4,1-4,16 (m,4H);δ 7,24-7,3 (m,6H); δ 7,77-7,84 (m,4H)

### b) Synthèse du polymère :

On porte au reflux, pendant 40 heures, un mélange de 7,31 g (0,025 mole) de 1,1'-(1,4-Butanediyl)bis-(benzimidazole), 50 ml de méthanol et 5,05 g (0,025 mole) de 1,3-dibromopropane. On concentre le mélange réactionnel, puis on reprend le résidu à l'éther isopropylique; après filtration et séchage, on obtient 12,3 g de poudre blanche % Br 31,65 (théorie 32,3%).

### 4) Synthèse de l'exemple 4 en solution aqueuse :

On porte à 110°C pendant 5 heures un mélange de 20,37 g (0,1 mole) de 1,1'-(1,4-Butanediyl)bis(imidazole) contenant 6,6% d'eau, 25 ml d'eau et 26,4 g (0,1 mole) de dibromo-α α' paraxylène dans un réacteur fermé hermétiquement. Le dosage par du nitrate d'argent d'une prise d'essai montre que 98,3% des bromures est sous forme ionique après 5 H de réaction. On ramène la solution à 50 % de matière active en tenant compte du taux de bromures.
Dosage des bromures par AgNO3 33,9% (théorie: 35,2%) sur un extrait sec.

### 5) Synthèse de l'exemple 5 en solution aqueuse :

On porte au reflux pendant 8 heures un mélange de 40,73 g (0,2 mole) de 1,1'-(1,4-Butanediyl)bis(imidazole) contenant 6,6% d'eau, 100 ml d'eau et 38,6 g (0,2 mole) de 1,2bis (2-Chloroethoxy) ethane Aldrich à 97%. Le dosage par du nitrate d'argent d'une prise d'essai montre que 97,8% des chlorures, après 8 heures de réaction, est sous forme ionique. On concentre légèrement le milieu réactionnel sous vide en présence de noir végétal, on filtre le noir et on ramène alors la solution à 50% de matière active en tenant compte du taux de chlorures. On obtient 127 g de solution légèrement ambrée.
Dosage des chlorures par AgNO₃ 18,5% (théorie : 18,8%) sur un extrait sec.

### 6) Synthèse de l'exemple 6 en solution aqueuse :

On porte au reflux pendant 21 heures, un mélange de 5,26 g à 95% (0,02 mole) de 1,1'(1,2 bis(ethoxy) ethanediyl)bis (imidazole), 10 ml d'eau et 3,74 g (0,02 mole) de 1,2 bis(2 chloroethoxy)ethane Aldrich à 97%. Le dosage par du nitrate d'argent d'une prise d'essai montre que 96% des chlorures, après 21 heures de réaction, est sous forme ionique. On ramène alors la solution à 50% de matière active en tenant compte du taux de chlorures. On obtient 15,5 g de solution jaune claire.

Dosage des chlorures par AgNO3 de la solution jaune clair: 2,28 meq/g.

### 7) Synthèse de l'exemple 7 en solution aqueuse :

On porte au reflux pendant 5 heures un mélange de 3,95 g (0,015 mole) de 1,1', 1,2 (bis(ethoxy)ethanedyil) bis(imidazole), 10 ml d'eau et 3,96 g (0,015 mole) de dibromo-αα' paraxylène. Le dosage par du nitrate d'argent d'une prise d'essai montre que 100% des bromures, après 5 heures de réaction, est sous forme ionique. On filtre la solution, la concentre légèrement sous vide, puis ramène la solution à 50% de matière active en tenant compte du taux de bromures. On obtient 14,8 g de solution jaune ambrée.

Dosage des bromures par AgNO3: 1,94 meq/g de solution.

### 8) Synthèse de l'exemple 8:

On porte au reflux pendant 10 heures un mélange de 2,38 g (0.01 mole) de 1,1'(αα' para xylène) bis imidazole, 9 ml d'eau et 2,6 g (0,01 mole) de dibromo -αα' para xylène. Le dosage par du nitrate d'argent d'une prise d'essai montre que 100% des bromures, après 10 heures de réaction, est sous forme ionique. On concentre le mélange réactionnel, puis on reprend le résidu dans 10 ml de méthanol; la poudre beige est filtrée. Après séchage sous vide, on obtient 3,6 g de poudre beige.

### 9) Synthèse de l'exemple 9 en solution aqueuse :

On porte au reflux pendant 5 heures un mélange de 20,37 g (0,1mole) de 1,1'-(1,4-Butanediyl)bis(imidazole) contenant 6,6% d'eau, 50 ml d'eau et 17,5 g (0,1 mole) de dichloro-αα' paraxylène. Le dosage par du nitrate d'argent d'une prise d'essai montre que 97% des chlorures, après 3 heures de réaction, est sous forme ionique, on laisse à 95°C encore pendant 2 heures. on ramène alors la solution à 50% de matière active en tenant compte du taux de chlorures. On obtient 68,4g de solution.

Dosage des chlorures par AgnO₃: 18,8% (théorie 19,4%) sur extrait sec.

### 10) Synthèse de l'exemple 10:

On porte au reflux pendant 122 heures un mélange de 15,94 g (0,1 mole) de 1H-imidazole-1-propanamine N,N-dimethyl à 96% ,40 ml de méthanol et 15,5 g (0,1 mole) de 1,6-dichlorohexane. Le dosage par du nitrate d'argent d'une prise d'essai montre que 96% des chlorures, après 122 heures de réaction, est sous forme ionique. On concentre la solution et on sèche le résidu sous vide de 0,1 mmHg. On obtient 30 g de poudre blanche très hygroscopique.

Dosage des chlorures par AgNO₃ : 22,0% (théorie: 22,9%).

### 11) Synthèse de l'exemple 11:

On porte au reflux pendant 5 heures un mélange de 2,51 g (0,0164 mole) de 1H-imidazole-1-propanamine N,N-dimethyl, 8,2 ml de méthanol et 2,87 g (0,0164 mole) de dichloro-αα' para xylène. Le dosage par du nitrate d'argent d'une prise d'essai montre que 94% des chlorures, après 5 heures de réaction, est sous forme ionique. On laisse à 95°C encore pendant 2 heures. On concentre la solution et on sèche le résidu sous vide. On obtient 5,4 g de poudre blanche très hygroscopique.

Dosage des chlorures par AgNO₃ : 19,5% (théorie : 21,6%).

### 12) Synthèse de l'exemple 12:

On porte au reflux pendant 6 heures un mélange de 6,95 g (0,036 mole) de 1-(1H-pipéridine)-3-(1H-imidazol)-propane, 15 ml de méthanol et 6,3 g (0,036 mole) de dichloro-αα' paraxylène. Le dosage par du nitrate d'argent d'une prise d'essai montre que 100% des chlorures, après 6 heures de réaction, est sous forme ionique. On concentre la solution et on sèche le résidu sous vide. On obtient 13 g de poudre légèrement rosée hygroscopique.

Dosage des chlorures par AgNO₃ : 18,3% (théorie: 19,2%).

### 13) Synthèse de l'exemple 13 :

On porte au reflux pendant 6 heures un mélange composé de 5,32 g (0,015 mole) de 1,1'-(1,4-Butanediyl)bis(phenyl-2-imidazole) à 96,6%, 10 ml d'eau et 3;24 g (0,015 mole) de dibromo-1,4 butane. Le dosage par du nitrate d'argent d'une prise d'essai montre que 100% des bromures, après 6 heures de réaction, est sous forme ionique. On filtre la solution, on la concentre légèrement sous vide, puis on ramène la solution à 50% de matière active en tenant compte du taux de bromures. On obtient 8 g de solution jaune clair. Dosage des bromures par AgNO₃: 1,79meq/g de solution.

### 14) Synthèse de l'exemple 14 :

On porte au reflux pendant 3,5 heures un mélange de 19,02g (0,1 mole) de 1,1'-(1,4-Butanediyl)bis(pyrazol), 40 ml d'eau et 17,5 g (0,1 mole) de dichloro-αα' -paraxylène. Le dosage par du nitrate d'argent d'une prise d'essai montre que 99% des chlorures, après 3,5 heures de réaction, sont sous forme ioniques. On ajoute environ 40ml d'eau, on filtre le léger trouble, on concentre et on amène la solution à 50% de matière active en tenant compte du taux de chlorures. On obtient une solution de 73g.
Dosage des chlorures par AgNO₃ : 17,96% (théorie: 19,4%) sur un extrait sec.

Les exemples suivants sont destinés à illustrer les compositions et l'application des polymères conformes à l'invention.

### EXEMPLE D'APPLICATION 1:

On prépare la composition suivante:
- Solution aqueuse de polymère de l'exemple 9 à 50% MA 3 g
- Acide thioglycolique 5 g
- Monoéthanolamine 2,6 g
- Parfum 0,5 g
- Alcool oléique oxyéthyléné (20 moles d'oxyde d'éthylène) 1 g
- Cocoyl amidopropyl diméthyl hydroxypropyl 2 g sulfobétaïne en solution aqueuse à 50%
- Acide diéthylène triamine pentacétique, sel 0,2 g pentasodique en solution aqueuse à 40%
- Eau déminéralisée qs 100 g

Cette composition est utilisée comme composition réductrice.

On applique cette composition sur des cheveux mouillés préalablement enroulés sur des rouleaux de mise en plis. Après avoir laissé agir la composition pendant environ 15 minutes, on rince abondamment à l'eau, puis on applique la composition oxydante suivante:
- Eau oxygénée qsp 8 volumes

On laisse agir la composition oxydante pendant environ 5 minutes, puis on rince abondamment la chevelure à l'eau et on enlève les rouleaux.

Après séchage, sous casque, les cheveux présentent de belles boucles.

### EXEMPLE D'APPLICATION 2:

On prépare la composition d'oxydation suivante:
- Solution aqueuse de polymère de l'exemple 4 à 50% MA 1 g
- Eau oxygénée à 50% 4,8 g
- Stabilisants 0,2 g
- Acide citrique qs pH 3
- Eau déminéralisée qs 100 g

On applique la composition réductrice suivante:
- Acide thioglycolique 5 g
- Monoéthanolamine 2,6 g
- Parfum 0,5 g
- Alcool oléique oxyéthyléné (20 moles d'oxyde d'éthylène) 1 g
- Cocoyl amidopropyl diméthyl hydroxyproyl 2 g sulfobétaïne en solution aqueuse à 50%
- Acide diéthylène triamine pentacétique, sel 0,2 g pentasodique en solution aqueuse à 40%
- Eau déminéralisée qs 100 g

On applique cette composition sur des cheveux mouillés préalablement enroulés sur des rouleaux de mise en plis. Après avoir laissé agir la composition pendant environ 15 minutes, on rince abondamment à l'eau, puis on applique la composition oxydante préparée ci-dessus:

On laisse agir la composition oxydante pendant environ 5 minutes, puis on rince abondamment la chevelure à l'eau et on enlève les rouleaux.

Après séchage, sous casque, les cheveux présentent de belles boucles.

### EXEMPLE D'APPLICATION 3:

On prépare la composition suivante:

| | |
|---|---|
| Octyldodécanol vendu sous la dénomination EUTANOL D par la société HENKEL | 8 g |
| Acide oléique | 20 g |
| Lauryléthersulfate de monoéthanolamine vendu sous la dénomination SIPON LM 35 par la société HENKEL | 3 g |
| Alcool éthylique | 10 g |
| Alcool benzylique | 10 g |
| Alcool cétylstéarylique à 33 moles d'oxyde d'éthylène vendu sous la dénomination SIMULSOL GS par la société SEPPIC | 2,4 g |
| Acide éthylène diamine tétracétique | 0,2 g |
| Solution aqueuse de polymère de l'exemple 4 à 50% M.A. | 3,7 g |
| Monoéthanolamine | 7,5 g |
| Diéthanolamide d'acide linoléique vendu sous la dénomination COMPERLAN F par la société HENKEL | 8 g |
| Ammoniaque à 20% de NH3 | 10,2 g |
| Métabisulfite de sodium en solution aqueuse à 35% | 1,3 g |
| Hydroquinone | 0,15 g |
| 1-phényl, 3-méthyl, 5-pyrazolone | 0,2 g |
| Paraphenylènediamine | 0,8 g |
| Métaaminophenol | 0,05 g |
| 2-méthyl 5-Nßhydroxyéthylaminophenol | 2,8 g |
| Dichlorhydrate de 2,4-diaminophenoxyethanol | 0,15 g |
| Eau déminéralisée q.s.p. | 100 g |

Cette composition est utilisée pour la teinture d'oxydation des cheveux.

La composition obtenue est mélangée, poids pour poids, avec de l'eau oxygénée titrant 20 volumes, et dont le pH est 3.
Le mélange ainsi réalisé est appliqué sur des cheveux gris à 90% de blancs, permanentés ou non, à raison de 28 g pour 3 g de cheveux, pendant 30 mn.
Les cheveux sont ensuite rincés, lavés avec un shampooing standard et séchés.
Les cheveux ont une coloration chatain cendré rouge (chatain violine) Ils sont doux au toucher, brillants et se démêlent facilement.

### EXEMPLE D'APPLICATION 4 :

On prépare la composition suivante:

| | |
|---|---|
| Alcool cétylstéarylique (C16/C18, 50/50) vendu sous la dénomination CIRE DE LANETTE O par la société HENKEL | 18 g |
| 2-octyl dodécanol | 3 g |
| Alcool cétylstéarylique (C16/C18, 35/65) oxyéthyléné (15 OE) vendu sous la dénomination MERGITAL CS 15 par la société SINNOVA - HENKEL | 3 g |
| Laurylsulfate d'ammonium à 30% M.A. | 12 g |
| Solution aqueuse de polymère de l'exemple 4 à 50% M.A. | 3 g |
| Thiolactate d'ammonium à 50% en équivalent d'acide thiolactique | 0,8 g |
| Ammoniaque à 20% de NH3 | 12 g |
| Paratoluylène diamine | 1,5 g |
| Résorcine | 0,6g |
| Métaaminophénol | 0,25 g |
| Paraaminophénol | 0,20 g |
| Dichlorhydrate de 2-Diaminophénoxyéthanol | 0,15 g |
| Eau déminéralisée q.s.p. | 100 g |

Cette composition se présente sous forme de crème.
La composition obtenue est diluée extemporanément avec 1,5 fois son volume d'eau oxygénée à 30 volumes, et dont le pH est 3.
Le mélange ainsi réalisé est appliqué sur des cheveux gris à 90% de blancs, permanentés ou non, à raison de 28 g pour 3 g de cheveux, pendant 30 mn. Les cheveux sont ensuite rincés, lavés avec un shampooing standard et séchés.
Les cheveux ont une coloration chatain foncé.
Ils sont doux au toucher, brillants et se démêlent facilement.

### EXEMPLE D'APPLICATION 5 :

On prépare la composition suivante:

| | |
|---|---|
| Octyldodécanol vendu sous la dénomination EUTANOL D par la société HENKEL | 8 g |
| Acide oléique | 20 g |
| Lauryléthersulfate de monoéthanolamine vendu sous la dénomination SIPON LM 35 par la société HENKEL | 3 g |
| Alcool éthylique | 10 g |
| Alcool benzylique | 10 g |
| Alcool cétylstéarylique à 33 moles d'oxyde d'éthylène vendu sous la dénomination SIMULSOL GS par la société SEPPIC | 2,4 g |
| Acide éthylène diamine tétracétique | 0,2 g |
| Solution aqueuse de polymère de l'exemple 4 à 50% M.A. | 3,7 g |
| Monoéthanolamine | 9,5 g |
| Diéthanolamide d'acide linoléique vendu sous la dénomination COMPERLAN F par la société HENKEL | 8 g |
| Métabisulfite de sodium en solution aqueuse à 35% | 1,3 g |
| Hydroquinone | 0,15 g |
| 1-phényl, 3-méthyl, 5-pyrazolone | 0,2 g |
| Paratoluylène diamine | 0,9 g |
| Résorcine | 0,2 g |
| Dichlorhydrate de 2-4 diaminophenoxyéthanol | 2,5 g |
| Eau déminéralisée q.s.p. | 100 g |

La composition obtenue est mélangée, poids pour poids, avec de l'eau oxygénée titrant 20 volumes, et dont le pH est 3.
Le mélange ainsi réalisé est appliqué sur des cheveux gris à 90% de blancs, permanentés ou non, à raison de 28 g pour 3 g de cheveux, pendant 30 mn.
Les cheveux sont ensuite rincés, lavés avec un shampoing standard et séchés.
Les cheveux ont une coloration noir bleuté intense.
Ils sont doux au toucher, brillants et se démêlent facilement.

## Revendications

1. Utilisation à titre d'agent protecteur des fibres kératiniques d'un polymère constitué à base des motifs récurrents de formule (I) : 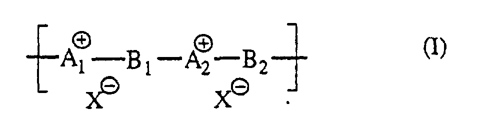 dans laquelle:
• A₁^{⊕}et A₂^{⊕}, identiques ou différents, désignent :
a) un hétérocycle insaturé quaternaire de formule (II): 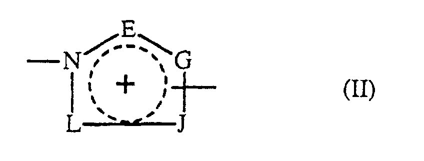 dans laquelle:
E, G, L, J, identiques ou différents, désignent un atome de carbone, d'oxygène, de soufre ou d'azote, l'un au moins désignant un atome d'azote;
E, G, L, J peuvent être substitués, lorsque un ou plusieurs de ces atomes désignent un atome de carbone, par un ou plusieurs atomes d'halogène, groupements hydroxyle, nitro, cyano, sulfhydryle, carboxyle, un groupement alkyle, monohydroxyalkyle, polyhydroxyalkyle, thioalkyle, cyanoalkyle, alkoxy, acyle, acétyloxyl, un groupement cycloalkyle substitué ou non, aryle substitué ou non, alkylaryle substitué ou non ou de groupements -NHR_{N} dans lequel R_{N} désigne un groupement acétyle ou uréido;
Lorsque E, G, L ou J désignent un troisième atome d'azote, celui-ci peut être substitué par un hydrogène, un radical alkyle, monohydroxy alkyle, polyhydroxy alkyle, aryle substitué ou non ou alkylaryle substitué ou non.
Les substituants de deux des atomes E, G, L et J peuvent également former conjointement avec les atomes auxquels ils sont rattachés un cycle aromatique de 5 à 7 chaînons substitué ou non; ou
b) un ammonium quaternaire de formule (III) 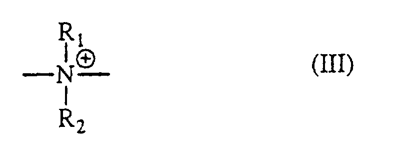 dans laquelle:
R₁ et R₂, identiques ou différents, désignent un groupement carboxyle, un groupement alkyle, polyhydroxyalkyle, thioalkyle, cyanoalkyle, alkoxy, acyle, acétyloxy, un groupement cycloalkyle substitué ou non, aryle substitué ou non, alkylaryle substitué ou non ou un groupemement -NHR_{N} dans lequel R_{N} désigne un groupement acétyle ou uréido;
R₁ et R₂ peuvent également former conjointement avec l'atome d'azote auquel ils sont rattachés un cycle saturé de 5 à 7 chaînons carbonés;
et dans laquelle au moins un des groupements A₁^{⊕}ou A₂^{⊕}désigne un hétérocycle insaturé de formule (II).
• B₁ et B₂, identiques ou différents, désignent un groupement hydrocarboné pouvant contenir, liés à ou intercalés dans la chaîne principale, un ou plusieurs cycles aromatiques substitués ou non, un ou plusieurs atomes d'oxygène, de soufre ou d'azote, un ou plusieurs groupements -SO-, -SO₂-, -SO₃H, amino, alkylamino, hydroxyle, ammonium quaternaire ou uréido.
• X^{⊖} représente un anion dérivé d'un acide organique ou minéral.

2. Utilisation selon la revendication 1, **caractérisée en ce qu'** au moins un des groupements A₁^{⊕} ou A₂^{⊕}, désigne un groupement imidazole quaternaire de formule (IV): 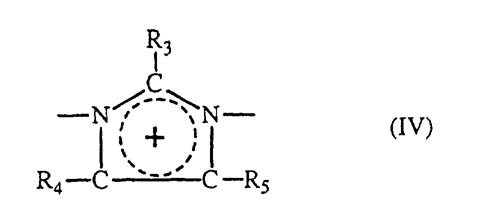 dans laquelle
R₃, R₄ et R₅, identiques ou différents, désignent un atome d'hydrogène, d'halogène, un groupement hydroxyle, nitro, cyano, sulfhydryle, carboxyle, un groupement alkyle, monohydroxyalkyle, polyhydroxyalkyle, thioalkyle, cyanoalkyle, alkoxy, acyle, acétyloxyl, un groupement cycloalkyle, aryle, alkylaryle ou un groupement -NHR_{N} dans lequel R_{N} désigne un atome d'hydrogène, un groupement alkyle, monohydroxyalkyle, polyhydroxyalkyle, acétyle ou uréido; les radicaux R₄ et R₅ peuvent également former conjointement avec les atomes auxquels ils sont rattachés un cycle aromatique de 5 à 7 chaînons.

3. Utilisation selon la revendication 1 ou 2, **caractérisée en ce qu'**au moins un des groupements A₁^{⊕}ou A₂^{⊕}, désigne un groupement pyrazole quaternaire de formule (V): 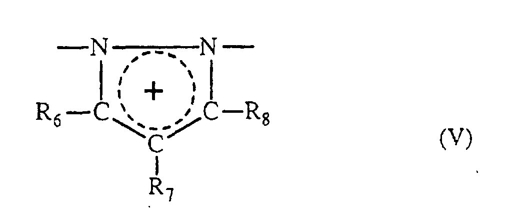 dans laquelle :
R₆, R₇ et R₈, identiques ou différents, désignent un atome d'hydrogène, d'halogène, un groupement hydroxyle, nitro, cyano, sulfhydryle, carboxyle, un groupement alkyle, monohydroxyalkyle, polyhydroxyalkyle, thioalkyle, cyanoalkyle, alkoxy, acyle, acétyloxyl, un groupement cycloalkyle, aryle, alkylaryle ou un groupement -NHR_{N} dans lequel R_{N} désigne un atome d'hydrogène, un groupement alkyle, monohydroxyalkyle, polyhydroxyalkyle, acétyle ou uréido;
deux des radicaux R₆, R₇ ou R₈ peuvent également former conjointement avec les atomes auxquels ils sont rattachés un cycle aromatique de 5 à 7 chaînons.

4. Utilisation selon la revendication 1 ou 2, **caractérisée en ce que** les groupements A₁^{⊕} et A₂^{⊕} désignent simultanément un groupement imidazole quaternaire de formule (IV), dans laquelle R₃, R₄ et R₅, identiques ou différents, désignent un atome d'hydrogène, un groupement hydroxyle, nitro, cyano, sulthydryle, carboxyle, un groupement alkyle, monohydroxyalkyle ou polyhydroxyalkyle; les radicaux R₄ et R₅ peuvent également former conjointement avec les atomes auxquels ils sont rattachés un cycle aromatique de 5 à 7 chaînons;
• B₁ et B₂, identiques ou différents, désignent un groupement polyméthylène pouvant contenir un ou plusieurs atomes d'oxygène et/ou un ou plusieurs cycles aromatiques.
• X^{⊖} est tel que défini dans la revendication 1.

5. Utilisation selon l'une quelconque des revendications 1, 2 ou 4, **caractérisée en ce que** A₁^{⊕} et A₂^{⊕} désignent simultanément un groupement imidazole quaternaire de formule (IV) dans laquelle:
• R₃, R₄ et R₅, identiques ou différents, désignent un atome d'hydrogène, un groupement alkyle en C₁ à C₆ et les radicaux R₄ et R₅ peuvent également former conjointement avec les atomes auxquels ils sont rattachés un cycle phényle.
• B₁ et B₂, identiques ou différents, désignent un groupement polyméthylène pouvant contenir un ou plusieurs atomes d'oxygène et/ou un ou plusieurs cycles aromatiques.
• X^{⊖} est tel que défini dans la revendication 1.

6. Utilisation selon la revendication 1 ou 2, **caractérisée en ce que** A₁ ^{⊕} représente un ammonium quaternaire de formule (III) dans laquelle R et R', identiques ou différents, désignent un atome d'hydrogène, ou un groupement alkyle en C₁ à C₆ ou forment conjointement un groupement cyclopentyle et A₂^{⊕} désigne un groupement imidazole quaternaire de formule (IV) dans laquelle R₃, R₄ et R₅, identiques ou différents, désignent un atome d'hydrogène, un alkyle en C₁-C₆ et les radicaux R₄ et R₅ peuvent également former conjointement avec les atomes auxquels ils sont rattachés un cycle aromatique de 5 à 7 chaînons.

7. Utilisation selon l'une quelconque des revendications 1 à 3,
**caractérisée en ce qu'**au moins un des groupements B₁ ou B₂ désigne un groupement de formule (VI):
―(CH₂)ₙ―CO―A―OC―(CH₂)ₙ― (VII)
dans laquelle:
n désigne un nombre entier de 1 à 10;
A désigne :
(a) un reste de glycol de formule: -O-Z-O-
dans laquelle Z désigne un groupement hydrocarboné, ou un groupement répondant aux formules: 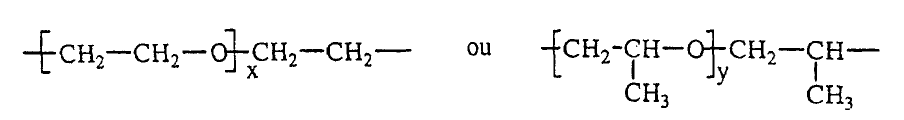 dans lesquelles x et y désignent un nombre quelconque de 1 à 4; ou
(b) un reste de diamine bis-secondaire; ou
(c) un reste de diamine bis-primaire de formule :
―NH―Y―NH―
dans laquelle Y désigne un groupement hydrocarboné ou le radical
―(CH₂)₂―S ― S ― (CH₂)₂ ― ;
ou
(d) un groupement uréylène

8. Utilisation selon l'une quelconque des revendications 1 à 7,
**caractérisée en ce que** B₁ et B₂, identiques ou différents, désignent un groupement de formule :
-(CH₂)ₙ - ,
n désignant un nombre entier de 1 à 6; ou 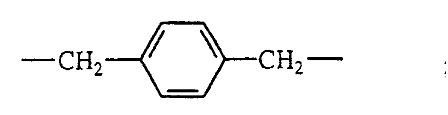 ou
― (CH₂)₂ ― O ― (CH₂)₂ ― O ― (CH₂)₂ ―

9. Utilisation selon l'une quelconque des revendications 1 à 8,
**caractérisée en ce que** X^{⊖} représente un anion halogénure.

10. Polymères constitués à base des motifs récurrents de formule (VII): 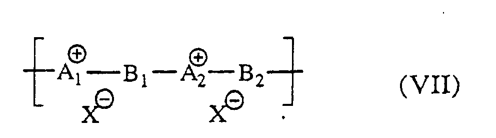 dans laquelle
A1^{⊕} désigne un groupement cationique insaturé de formule (II) et
A2^{⊕} désigne un ammonium quaternaire de formule (III);
B₁, B₂, X^{⊖} et les formules (II) et (III) étant tels que définis dans la revendication 1, et au moins un des groupements B₁ ou B₂ est différent de -CH₂-CHOH-CH₂-.

11. Polymères constitués, à base des motifs récurrents de formule (VIII) : 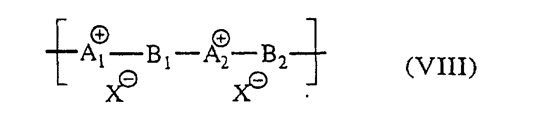 dans laquelle:
A₁^{⊕} désigne un groupement imidazole quaternaire de formule (IV) et A₂^{⊕} désigne un groupement pyrazole quaternaire de formule (V); B₁, B₂, X^{⊖}, les formules (IV) et (V) sont tels que définis dans les revendications 1, 2 et 3.

12. Polymères constitués, à base des motifs récurrents de formule (IX) : 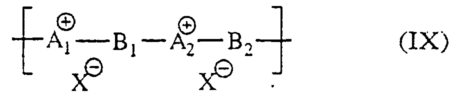 dans laquelle:
A₁^{⊕} est tel que défini dans la revendication 1, et dans laquelle A₂^{⊕} désigne un groupement imidazole quaternaire de formule (IV) telle que définie dans la revendication 2 dans laquelle R₄ et R₅, identiques ou différents, désignent un atome d'hydrogène, d'halogène, un groupement hydroxyle, nitro, cyano, sulfhydryle, carboxyle, un groupement alkyle, monohydroxyalkyle,
polyhydroxyalkyle, thioalkyle, cyanoalkyle, alkoxy, acyle, acétyloxyl, un groupement cycloalkyle, aryle, alkylaryle ou un groupement -NHR_{N} dans lequel R_{N} désigne un atome d'hydrogène, un groupement alkyle, monohydroxyalkyle, polyhydroxyalkyle, acétyle ou uréido et R₃ désigne un groupement cycloalkyl, aryle ou alkylaryle; B₁, B₂ et X^{⊖} sont tels que définis dans la revendication 1.

13. Composition réductrice pour la mise en forme permanente des fibres kératiniques, **caractérisée par le fait qu'**elle contient dans un milieu approprié pour la permanente, au moins un agent réducteur et au moins un polymère tel que définis dans l'une quelconque des revendications 1 à 12.

14. Composition selon la revendication 13, **caractérisée par le fait que** l'agent réducteur est choisi parmi les sulfites, les bisulfites et les thiols.

15. Composition réductrice selon la revendication 13 ou 14, **caractérisée par le fait que** les agents réducteurs sont choisis parmi la cystéine, la cystéamine et leurs sels cosmétiquement acceptables, l'acide thiolactique, l'acide thioglycolique et leurs esters cosmétiquement acceptables.

16. Composition réductrice selon l'une quelconque des revendications 13 à 15, **caractérisée par le fait qu'**elle contient 0,01 à 10% en poids de polymère tel que défini dans l'une quelconque des revendications 1 à 12.

17. Composition oxydante pour la mise en forme permanente des fibres kératiniques, **caractérisée par le fait qu'**elle contient dans un milieu approprié pour la permanente, au moins un agent oxydant et au moins un polymère tel que défini dans l'une quelconque des revendications 1 à 12.

18. Composition oxydante selon la revendication 17, **caractérisée par le fait que** l'agent oxydant est choisi parmi l'eau oxygénée, les peroxydes d'urée, les bromates tels que les bromates alcalins, les persels ou un mélange de bromates alcalins et d'un persel.

19. Composition oxydante selon la revendication 17 ou 18, **caractérisée par le fait qu'**elle contient de 0,01 à 10% en poids d'un polymère tel que défini dans l'une quelconque des revendications 1 à 12.

20. Composition pour la teinture directe des fibres kératiniques et en particulier des fibres kératiniques humaines telles que les cheveux, **caractérisée par le fait qu'**elle comprend, dans un milieu approprié pour la teinture, au moins un polymère tel que défini dans l'une quelconque des revendications 1 à 12 et au moins un colorant direct.

21. Composition selon la revendication 20, **caractérisée par le fait que** le colorant direct est choisi parmi les colorants azoïques, anthraquinoniques et les dérivés nitrés de la série benzénique

22. Composition pour la teinture d'oxydation des fibres kératiniques, en particulier des fibres kératiniques humaines tels que les cheveux, **caractérisée par le fait qu'**elle comprend, dans un milieu approprié pour la teinture, au moins un polymère tel que défini dans l'une quelconque des revendications 1 à 12 et au moins un précurseur de colorant d'oxydation et/ou un précurseur de mélanine.

23. Composition selon la revendication 22, **caractérisée par le fait que** les précurseurs de colorants d'oxydation sont choisis parmi les paraphénylène diamines, les paraaminophénols, les orthophénylènes diamines et les bases hétérocycliques.

24. Composition selon la revendication 22 ou 23, **caractérisée par le fait qu'**elle contient en outre des coupleurs.

25. Composition selon la revendication 24, **caractérisée par le fait que** les coupleurs sont choisis parmi les métaphénylènes diamines, les métaaminophénols, les metadiphénols, les coupleurs heterocycliques, les dérivés indoliniques, les dérivés de benzomidazole, les dérivés de benzomorpholines, les dérivés de sésamol, les dérivés pyridiniques, pyrimidiniques et pyrazoliques et leurs sels d'addtion avec un acide.

26. Composition tinctoriale selon l'une quelconque des revendications 20 à 25, **caractérisée par le fait qu'**elle contient de 0,01% à 10% en poids d'un polymère tel que défini dans l'une quelconque des revendications 1 à 12.

27. Composition d'oxydation pour la teinture d'oxydation des fibres kératiniques, **caractérisée par le fait qu'**elle contient dans un milieu approprié pour la teinture, au moins un agent oxydant et au moins un polymère tel que défini dans l'une quelconque des revendications 1 à 12.

28. Composition d'oxydation selon la revendication 27, **caractérisée par le fait que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à deux électrons.

29. Composition de décoloration, **caractérisée par le fait qu'**elle contient, dans un milieu approprié pour la décoloration, un agent décolorant les cheveux et un polymère tel que défini dans l'une quelconque des revendications 1 à 12.

30. Composition selon la revendication 29, **caractérisée par le fait que** l'agent décolorant est choisi parmi le peroxyde d'hydrogène, les persulfates, les percarbonates de sodium, les perborates.

31. Procédé de coloration des cheveux, **caractérisé par le fait que** l'on applique sur les cheveux, dans une quantité suffisante pour teindre les cheveux, une composition contenant un précurseur de colorant, diluée au moment de l'emploi avec une composition oxydante, qu'on laisse agir la composition pendant une durée comprise entre 5 et 45 minutes environ et qu'on rince les cheveux; la composition contenant le précurseur et/ou la composition oxydante étant respectivement telle que définie dans l'une quelconque des revendications 22 à 26 et telle que définie dans l'une des revendications 27 ou 28.

32. Procédé de coloration des cheveux, **caractérisé par le fait que** l'on applique sur les cheveux, dans une quantité suffisante pour produire une coloration, la composition telle que définie dans la revendication 20, 21 ou 22 qu'on laisse agir la composition pendant une durée comprise entre 10 et 60 minutes environ et qu'on rince éventuellement les cheveux.

33. Procédé de décoloration des cheveux, **caractérisé par le fait que** l'on applique sur les cheveux la composition décolorante définie dans l'une quelconque des revendications 29 et 30, dans une quantité et pendant une durée suffisante pour décolorer les cheveux et qu'on rince ensuite.

34. Procédé de mise en forme permanente des fibres kératiniques, **caractérisé par le fait que** l'on applique sur les fibres kératiniques une composition réductrice, qu'après un temps de pose suffisant pour réduire la kératine, on applique une composition oxydante et qu'on procède au rinçage après un temps de pose suffisant pour fixer les fibres kératiniques sous une forme permanente; la composition réductrice et/ou la composition oxydante étant respectivement telle que définie dans l'une quelconque des revendications 13 à 16 et telle que définie dans l'une quelconque des revendications 17 à 19.

35. Composition destinée au traitement cosmétique des cheveux, **caractérisée par le fait qu'**elle contient au moins un polymère tel que défini dans l'une quelconque des revendications 10 à 12.

## Patentansprüche

1. Verwendung eines Polymers, das auf wiederkehrenden Einheiten der Formel (I) basiert, als Schutzmittel für Keratinfasern: 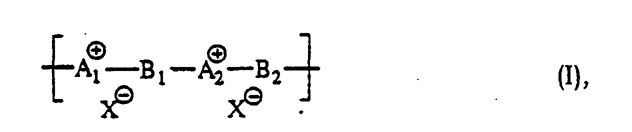 worin bedeuten:
• die Gruppen A₁^{⊕} und A₂^{⊕}, die gleich oder verschieden sind:
a) einen quartären ungesättigten Heterocyclus der Formel (II): 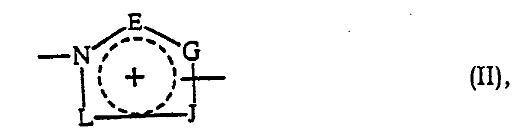 worin bedeuten:
die Atome E, G, L und J, die identisch oder voneinander verschieden sind, ein Kohlenstoffatom, ein Sauerstoffatom, ein Schwefelatom oder ein Stickstoffatom, wobei mindestens ein Atom ein Stickstoffatom bedeutet;
wenn ein oder mehrere der Atome E, G, L und J ein Kohlenstoffatom bedeuten, können diese Atome mit einem oder mehreren Halogenatomen oder einer oder mehreren der folgenden Gruppen substituiert sein: Hydroxy, Nitro, Cyano, Sulfhydryl, Carboxy, Alkyl, Monohydroxyalkyl, Polyhydroxyalkyl, Thioalkyl, Cyanoalkyl, Alkoxy, Acyl, Acetyloxy, Cycloalkylgruppen, die gegebenenfalls substituiert sind, Arylgruppen, die gegebenenfalls substituiert sind, Alkylarylgruppen, die gegebenenfalls substituiert sind oder -NHR_{N}, worin R_{N} eine Acetyl- oder Ureidogruppe bedeutet; wenn E, G, L oder J ein drittes Stickstoffatom bedeutet, kann dieses mit Wasserstoff, Alkyl, Monohydroxyalkyl, Polyhydroxyalkyl, einer gegebenenfalls substituierten Arylgruppe oder einer gegebenenfalls substituierten Alkylarylgruppe substituiert sein;
die Substituenten an zwei Atomen E, G, L und J können auch gemeinsam mit den Atomen, an die sie gebunden sind, einen aromatischen, gegebenenfalls substituierten 5-bis 7-gliedrigen Ring bilden; oder
b) eine quartäre Ammoniumgruppe der Formel (III) 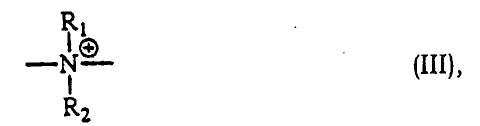 worin bedeuten:
R₁ und R₂, die identisch oder voneinander verschieden sind, Carboxy, Alkyl, Polyhydroxyalkyl, Thioalkyl, Cyanoalkyl, Alkoxy, Acyl, Acetyloxy, gegebenenfalls substituierte Cycloalkylgruppen, gegebenenfalls substituierte Arylgruppen, gegebenenfalls substituierte Alkylarylgruppen oder -NHR_{N}, worin die Gruppe R_{N} Acetyl oder Ureido bedeutet;
R₁ und R₂ können auch gemeinsam mit dem Stickstoffatom, an das sie gebunden sind, einen gesättigten Ring mit 5 bis 7 Kohlenstoffgliedern bilden;
wobei mindestens eine Gruppe A₁^{⊕} oder A₂^{⊕} einen ungesättigten quartären Heterocyclus der Formel (II) bedeutet;
• die Gruppen B₁ und B₂, die gleich oder verschieden sind, eine Kohlenwasserstoffgruppe, die an die Hauptkette gebunden oder in der Hauptkette einen oder mehrere substituierte oder unsubstituierte aromatische Ringe, ein oder mehrere Sauerstoffatome, Schwefelatome oder Stickstoffatome, eine oder mehrere Gruppen -SO-, -SO₂-, -SO₃H, Amino, Alkylamino, Hydroxy, quartäres Ammonium oder Ureido enthalten können;
• X^{⊖} ein Anion, das von einer organischen oder anorganischen Säure abgeleitet ist.

2. Verwendung nach Anspruch 1, **dadurch gekennzeichnet, dass** mindestens eine der Gruppen A₁^{⊕} und A₂^{⊕} eine quartäre Imidazolgruppe der Formel (IV) bedeutet: 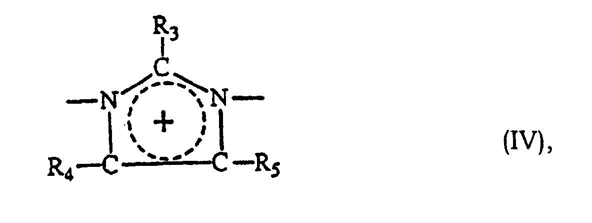 worin bedeuten:
die Gruppen R₃, R₄ und R₅, die gleich oder verschieden sind, Wasserstoff, Halogen, Hydroxy, Nitro, Cyano, Sulfhydryl, Carboxy, Alkyl, Monohydroxyalkyl, Polyhydroxyalkyl, Thioalkyl, Cyanoalkyl, Alkoxy, Acyl, Acetyloxy, Cycloalkyl, Aryl, Alkylaryl oder eine Gruppe -NHR_{N}, worin R_{N} Wasserstoff, Alkyl, Monohydroxyalkyl, Polyhydroxyalkyl, Acetyl oder Ureido bedeutet;
die Gruppen R₄ und Rs können auch gemeinsam mit den Atomen, an die sie gebunden sind, einen 5- bis 7-gliedrigen aromatischen Ring bilden.

3. Verwendung nach Anspruch 1 oder 2, **dadurch gekennzeichnet, dass** mindestens eine der Gruppen A₁^{⊕} oder A₂^{⊕} eine quartäre Pyrazolgruppe der Formel (V) bedeutet: 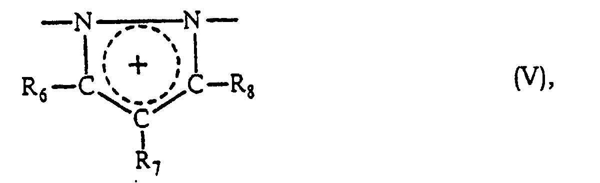 worin bedeuten:
R₆, R₇ und R₈, die gleich oder verschieden sind, Wasserstoff, Halogen, Hydroxy, Nitro, Cyano, Sulfhydryl, Carboxy, Alkyl, Monohydroxyalkyl, Polyhydroxyalkyl, Thioalkyl, Cyanoalkyl, Alkoxy, Acyl, Acetyloxy, Cycloalkyl, Aryl, Alkylaryl oder eine Gruppe -NHR_{N}, worin R_{N} Wasserstoff, Alkyl, Monohydroxyalkyl, Polyhydroxyalkyl, Acetyl oder Ureido bedeutet; zwei der Gruppen R₆, R₇ oder R₈ können auch gemeinsam mit den Atomen, an die sie gebunden sind, einen 5- bis 7-gliedrigen aromatischen Ring bilden.

4. Verwendung nach Anspruch 1 oder 2, **dadurch gekennzeichnet, dass** die Gruppen A₁^{⊕} und A₂^{⊕} gleichzeitig eine quartäre Imidazolgruppe der Formel (IV) bedeuten, worin die Gruppen R₃, R₄ und R₅, die gleich oder verschieden sind, Wasserstoff, Hydroxy, Nitro, Cyano, Sulfhydryl, Carboxy, Alkyl, Monohydroxyalkyl oder Polyhydroxyalkyl bedeuten; wobei die Gruppen R₄ und Rs auch gemeinsam mit den Atomen, an die sie gebunden sind, einen 5-bis 7-gliedrigen aromatischen Ring bilden können;
• die Gruppen B₁ und B₂, die gleich oder verschieden sind, bedeuten eine Polymethylengruppe, die ein oder mehrere Sauerstoffatome und/oder einen oder mehrere aromatische Ringe enthalten kann;
• X^{⊖} hat die in Anspruch 1 angegebenen Bedeutungen.

5. Verwendung nach einem der Ansprüche 1, 2 oder 4, **dadurch gekennzeichnet, dass** A₁^{⊕} und A₂^{⊕} gleichzeitig eine quartäre Imidazolgruppe der Formel (IV) sind, worin bedeuten:
• R₃, R₄ und R₅, die gleich oder verschieden sind, ein Wasserstoffatom oder eine C₁₋₆-Alkylgruppe, wobei die Gruppen R₄ und R₅ auch gemeinsam mit den Atomen, an die sie gebunden sind, eine Phenylgruppe bilden können;
• B₁ und B₂, die gleich oder verschieden sind, eine Polymethylengruppe, die ein oder mehrere Sauerstoffatome und/oder einen oder mehrere aromatische Ringe enthalten kann;
• X^{⊖} die in Anspruch 1 angegebenen Bedeutungen.

6. Verwendung nach Anspruch 1 oder 2, **dadurch gekennzeichnet, dass** A₁^{⊕} eine quartäre Ammoniumgruppe der Formel (III) bedeutet, worin die Gruppen R und R', die identisch oder voneinander verschieden sind, ein Wasserstoffatom oder eine C₁₋₆-Alkylgruppe bedeuten oder gemeinsam eine Cyclopentylgruppe bilden, und A₂^{⊕} eine quartäre Imidazolgruppe der Formel (IV) ist, worin die Gruppen R₃, R₄ und R₅, die gleich oder verschieden sind, Wasserstoff oder eine C₁₋₆-Alkylgruppe bedeuten, wobei die Gruppen R₄ und R₅ auch gemeinsam mit den Atomen, an die sie gebunden sind, einen 5- bis 7-gliedrigen aromatischen Ring bilden können.

7. Verwendung nach einem der Ansprüche 1 bis 3, **dadurch gekennzeichnet, dass** mindestens eine der Gruppen B₁ oder B₂ eine Gruppe der Formel (VI) bedeutet:
―(CH₂)ₙ―CO―A―OC―(CH₂)ₙ― (VI),
worin bedeuten:
n eine ganze Zahl von 1 bis 10,
A:
(a) eine Glykolgruppe der Formel ―O―Z―O―, worin Z eine Kohlenwasserstoffgruppe oder eine Gruppe der folgenden Formeln bedeutet: 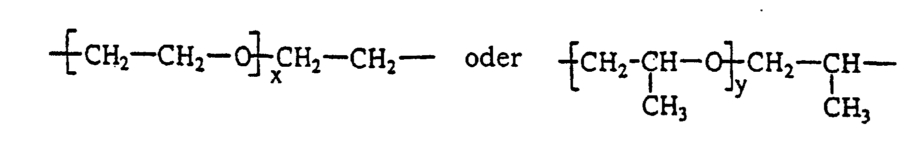 worin x und y beliebige Zahlen von 1 bis 4 bedeuten; oder
(b) eine bis-sekundäre Diamingruppe, wie ein Piperazinderivat; oder
(c) eine bis-primäre Diamingruppe der Formel:
―NH―Y―NH―,
worin Y eine Kohlenwasserstoffgruppe oder eine Gruppe
―(CH₂)₂―S―S―(CH₂)₂―
bedeutet; oder
(d) eine Ureylengruppe.

8. Verwendung nach einem der Ansprüche 1 bis 7, **dadurch gekennzeichnet, dass** die Gruppen B₁ und B₂, die gleich oder verschieden sind, eine Gruppe der folgenden Formel bedeuten:
―(CH₂)ₙ―, wobei n eine ganze Zahl von 1 bis 6 ist; oder 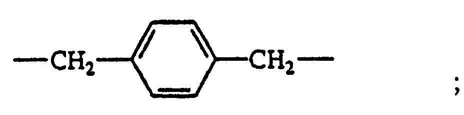 oder
―(CH₂)₂―O―(CH₂)₂―O―(CH₂)₂―,

9. Verwendung nach einem der Ansprüche 1 bis 8, **dadurch gekennzeichnet, dass** X^{⊖} ein Halogenid ist.

10. Polymere, die auf wiederkehrenden Einheiten der Formel (VII) basieren: 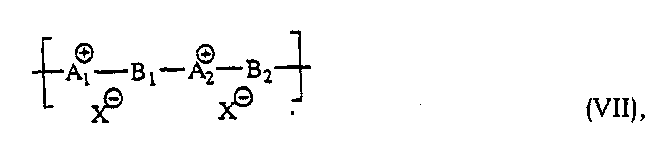 worin bedeuten:
A₁^{⊕} eine ungesättigte kationische Gruppe der Formel (II) und A₂^{⊕} eine quartäre Ammoniumgruppe der Formel (III);
wobei B₁, B₂ und X^{⊖} und die Formeln (II) und (III) die in Anspruch 1 angegebenen Bedeutungen aufweisen und wobei mindestens eine der Gruppen B₁ oder B₂ von ―CH₂―CHOH―CH₂ verschieden ist.

11. Polymere, die auf wiederkehrenden Einheiten der Formel (VIII) basieren: 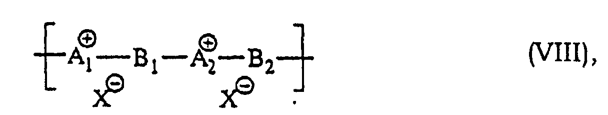 worin bedeuten:
A₁^{⊕} eine quartäre Imidazolgruppe der Formel (IV) und A₂^{⊕} eine quartäre Pyrazolgruppe der Formel (V); wobei die Gruppen B₁,
B₂ und X^{⊖} und die Formeln (IV) und (V) die in den Ansprüche 1, 2 und 3 angegebenen Bedeutungen aufweisen.

12. Polymere, die auf wiederkehrenden Einheiten der Formel (IX) basieren: 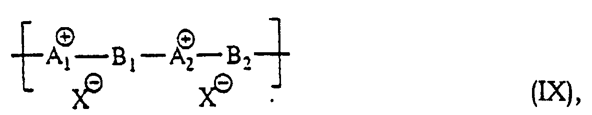 worin bedeuten:
A₁^{⊕} die in Anspruch 1 angegebenen Bedeutungen und A₂^{⊕} eine quartäre Imidazolgruppe der Formel (IV) wie in Anspruch 2 definiert, wobei R₄ und R₅, die gleich oder verschieden sind, Wasserstoff, Halogen, Hydroxy, Nitro, Cyano, Sulfhydryl, Carboxy, Alkyl, Monohydroxyalkyl, Polyhydroxyalkyl, Thioalkyl, Cyanoalkyl, Alkoxy, Acyl, Acetyloxy, Cycloalkyl, Aryl, Alkylaryl oder eine Gruppe -NHR_{N} bedeuten, worin R_{N} Wasserstoff, Alkyl, Monohydroxyalkyl, Polyhydroxyalkyl, Acetyl oder Ureido bedeutet, und worin R₃ Cycloalkyl, Aryl oder Alkylaryl ist; wobei B₁, B₂ und X^{⊖} die in Anspruch 1 angegebenen Bedeutungen aufweisen.

13. Reduzierende Zusammensetzung zur dauerhaften Verformung von Keratinfasern, **dadurch gekennzeichnet, dass** sie in einem zur dauerhaften Verformung geeigneten Medium mindestens ein Reduktionsmittel und mindestens ein Polymer nach einem der Ansprüche 1 bis 12 enthält.

14. Zusammensetzung nach Anspruch 13, **dadurch gekennzeichnet, dass** das Reduktionsmittel unter den Sulfiten, Bisulfiten und Thiolen ausgewählt ist.

15. Reduzierende Zusammensetzung nach Anspruch 13 oder 14, **dadurch gekennzeichnet, dass** die Reduktionsmittel unter Cystein, Cysteamin und deren kosmetisch akzeptablen Salzen, Thiomilchsäure, Thioglykolsäure und deren kosmetisch akzeptablen Estern ausgewählt sind.

16. Reduzierende Zusammensetzung nach einem der Ansprüche 13 bis 15, **dadurch gekennzeichnet, dass** sie 0,01 bis 10 Gew.-% Polymer nach einem der Ansprüche 1 bis 12 enthält.

17. Oxidierende Zusammensetzung zur dauerhaften Verformung von Keratinfasern, **dadurch gekennzeichnet, dass** sie in einem für die dauerhafte Verformung geeigneten Medium mindestens ein Oxidationsmittel und mindestens ein Polymer nach einem der Ansprüche 1 bis 12 enthält.

18. Oxidierende Zusammensetzung nach Anspruch 17, **dadurch gekennzeichnet, dass** das Oxidationsmittel unter Wasserstoffperoxid, Harnstoffperoxid, Bromaten, wie Alkalibromaten, Salzen von Persäuren oder Gemischen von Alkalibromaten und Salzen von Persäuren ausgewählt ist.

19. Oxidierende Zusammensetzung nach Anspruch 17 oder 18, **dadurch gekennzeichnet, dass** sie 0,01 bis 10 Gew.-% eines Polymers nach einem der Ansprüche 1 bis 12 enthält.

20. Zusammensetzung zur direkten Färbung von Keratinfasern und insbesondere menschlichen Keratinfasern, wie dem Haar, **dadurch gekennzeichnet, dass** sie in einem zum Färben geeigneten Medium mindestens ein Polymer nach einem der Ansprüche 1 bis 12 und mindestens einen Direktfarbstoff enthält.

21. Zusammensetzung nach Anspruch 20, **dadurch gekennzeichnet, dass** der Direktfarbstoff unter den Azofarbstoffen, Anthrachinon-Farbstoffen und den nitrierten Derivaten aus der Benzolgruppe ausgewählt ist.

22. Zusammensetzung zum oxidativen Färben von Keratinfasern und insbesondere menschlichen Keratinfasern, wie dem Haar, **dadurch gekennzeichnet, dass** sie in einem zum Färben geeigneten Medium mindestens ein Polymer nach einem der Ansprüche 1 bis 12 und mindestens ein Farbstoffvorprodukt eines Oxidationsfarbstoffes und/oder einen Melanin-Vorläufer enthält.

23. Zusammensetzung nach Anspruch 22, **dadurch gekennzeichnet, dass** die Farbstoffvorprodukte von Oxidationsfarbstoffen unter den p-Phenylendiaminen, p-Aminophenolen, o-Phenylendiaminen und den heterocyclischen Basen ausgewählt sind.

24. Zusammensetzung nach Anspruch 22 oder 23, **dadurch gekennzeichnet, dass** sie ferner Kuppler enthält.

25. Zusammensetzung nach Anspruch 24, **dadurch gekennzeichnet, dass** die Kuppler unter den m-Phenylendiaminen, m-Aminophenolen, m-Dihydroxybenzolen, heterocyclischen Kupplern, Indolinderivaten, Benzimidazolderivaten, Benzomorpholinderivaten, Sesamolderivaten, Pyridinderivaten, Pyrimidinderivaten, Pyrazolderivaten und deren Additionssalzen mit einer Säure ausgewählt sind.

26. Farbmittelzusammensetzung nach einem der Ansprüche 20 bis 25, **dadurch gekennzeichnet, dass** sie 0,01 bis 10 Gew.-% Polymer nach einem der Ansprüche 1 bis 12 enthält.

27. Oxidierende Zusammensetzung zum oxidativen Färben von Keratinfasern, **dadurch gekennzeichnet, dass** sie in einem zum Färben geeigneten Medium mindestens ein Oxidationsmittel und mindestens ein Polymer nach einem der Ansprüche 1 bis 12 enthält.

28. Oxidierende Zusammensetzung nach Anspruch 27, **dadurch gekennzeichnet, dass** das Oxidationsmittel unter Wasserstoffperoxid, Harnstoffperoxid, Bromaten von Alkalimetallen, Salzen von Persäuren, wie Perboraten und Persulfaten und den Enzymen, wie Peroxidasen und Oxidoreductasen (zwei Elektronen), ausgewählt ist.

29. Zusammensetzung zum Entfärben, **dadurch gekennzeichnet, dass** sie in einem zum Entfärben geeigneten Medium ein Mittel zum Entfärben der Haare und ein Polymer nach einem der Ansprüche 1 bis 12 enthält.

30. Zusammensetzung nach Anspruch 29, **dadurch gekennzeichnet, dass** das Mittel zum Entfärben unter Wasserstoffperoxid, Persulfaten, Natriumpercarbonaten und Perboraten ausgewählt ist.

31. Verfahren zum Färben der Haare, **dadurch gekennzeichnet, dass** auf die Haare in einer zum Färben der Haare ausreichenden Menge eine Zusammensetzung aufgebracht wird, die ein Farbstoffvorprodukt eines Farbstoffes enthält und die bei der Anwendung mit einer oxidierenden Zusammensetzung vermischt wird, die Zusammensetzung während einer Zeitdauer im Bereich von etwa 5 bis 45 Minuten einwirken gelassen wird, worauf die Haare gespült werden; wobei die Zusammensetzung, die das Farbstoffvorsprodukt enthält, und/oder die oxidierende Zusammensetzung Zusammensetzungen nach einem der Ansprüche 22 bis 26 und nach einem der Ansprüche 27 oder 28 sind.

32. Verfahren zum Färben der Haare, **dadurch gekennzeichnet, dass** auf die Haare in einer zum Färben ausreichenden Menge eine Zusammensetzung nach einem der Ansprüche 20, 21 oder 22 aufgebracht wird, die Zusammensetzung während einer Zeitdauer von etwa 10 bis 60 Minuten einwirken gelassen und die Haare gegebenenfalls gespült werden.

33. Verfahren zum Entfärben der Haare, **dadurch gekennzeichnet, dass** auf die Haare die entfärbende Zusammensetzung nach einem der Ansprüche 29 und 30 in einer Menge und während einer Zeitspanne aufgebracht wird, die zum Entfärben der Haare ausreichend ist, worauf anschließend gespült wird.

34. Verfahren zur dauerhaften Verformung von Keratinfasern, **dadurch gekennzeichnet, dass** auf die Keratinfasern eine reduzierende Zusammensetzung aufgetragen wird, nach einer zur Reduktion des Keratins ausreichenden Zeitspanne eine oxidierende Zusammensetzung aufgetragen wird und nach einer zur Fixierung der Keratinfasern in einer permanenten Form ausreichenden Zeitspanne gespült wird; wobei die reduzierende Zusammensetzung und/oder die oxidierende Zusammensetzung Zusammensetzungen nach einem der Ansprüche 13 bis 16 und nach einem der Ansprüche 17 bis 19 sind.

35. Zusammensetzung zur kosmetischen Behandlung der Haare, **dadurch gekennzeichnet, dass** sie mindestens ein Polymer nach einem der Ansprüche 10 bis 12 enthält.

## Claims

1. Use, as protective agent for keratinous fibres, of a polymer basically composed of repeat units of formula (I): 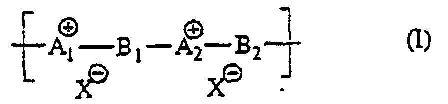 in which:
• A₁^{⊕} and A₂^{⊕}, which are identical or different, denote:
a) an unsaturated quaternary heterocycle of formula (II): 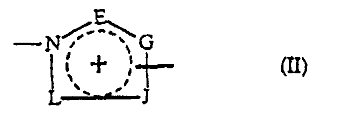 in which:
E, G, L and J, which are identical or different, denote a carbon, oxygen, sulphur or nitrogen atom, at least one denoting a nitrogen atom;
E, G, L and J can be substituted, when one or more of these atoms denote a carbon atom, by one or more halogen atoms, hydroxyl, nitro, cyano, mercapto or carboxyl groups, an alkyl, monohydroxyalkyl, polyhydroxyalkyl, thioalkyl, cyanoalkyl, alkoxy, acyl or acetyloxy group, a substituted or unsubstituted cycloalkyl, substituted or unsubstituted aryl, or substituted or unsubstituted alkylaryl group, or -NHR_{N} groups in which R_{N} denotes an acetyl or ureido group.
When E, G, L or J denotes a third nitrogen atom, the latter can be substituted by hydrogen or an alkyl, monohydroxyalkyl, polyhydroxyalkyl, substituted or unsubstituted aryl, or substituted or unsubstituted alkylaryl radical.
The substituents of two of the E, G, L and J atoms can also form, jointly with the atoms to which they are attached, a substituted or unsubstituted 5- to 7-membered aromatic ring; or
b) a quaternary ammonium of formula (III) 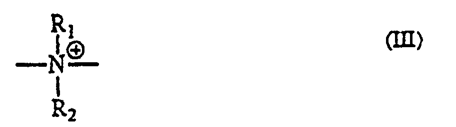 in which:
R₁ and R₂, which are identical or different, denote a carboxyl group, an alkyl, polyhydroxyalkyl, thioalkyl, cyanoalkyl, alkoxy, acyl or acetyloxy group, a substituted or unsubstituted cycloalkyl, substituted or unsubstituted aryl or substituted or unsubstituted alkylaryl group or an -NHR_{N} group in which R_{M} denotes an acetyl or ureido group;
R₁ and R₂ can also form, jointly with the nitrogen atom to which they are attached, a saturated 5- to 7-membered carbonaceous ring;
and in which at least one of the A₁^{⊕} or A₂^{⊕} groups denotes an unsaturated quaternary heterocycle of formula (II).
• B₁ and B₂, which are identical or different, denote a hydrocarbonaceous group which can comprise, bonded to or inserted in the main chain, one or more substituted or unsubstituted aromatic rings, one or more oxygen, sulphur or nitrogen atoms, or one or more -SO-, -SO₂-, -SO₃H, amino, alkylamino, hydroxyl, quaternary ammonium or ureido groups.
• X^{⊖} represents an anion derived from an organic or inorganic acid.

2. Use according to Claim 1, **characterized in that** at least one of the A₁^{⊕} or A₂^{⊕} groups denotes a quaternary imidazole group of formula (IV): 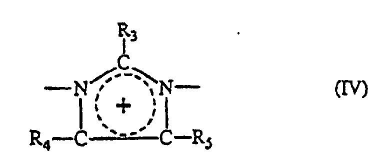 in which
R₃, R₄ and R₅, which are identical or different, denote a hydrogen or halogen atom, a hydroxyl, nitro, cyano, mercapto or carboxyl group, an alkyl, monohydroxyalkyl, polyhydroxyalkyl, thioalkyl, cyanoalkyl, alkoxy, acyl or acetyloxy group, a cycloalkyl, aryl or alkylaryl group or an -NHR_{N} group in which R_{N} denotes a hydrogen atom or an alkyl, monohydroxyalkyl, polyhydroxyalkyl, acetyl or ureido group;
the R₄ and R₅ radicals can also form, jointly with the atoms to which they are attached, a 5- to 7-membered aromatic ring.

3. Use according to Claim 1 or 2, **characterized in that** at least one of the A₁^{⊕} or A₂^{⊕} groups denotes a quaternary pyrazole group of formula (V): 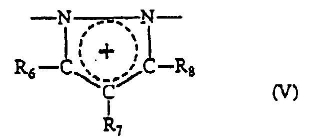 in which:
R₆, R₇ and R₈, which are identical or different, denote a hydrogen or halogen atom, a hydroxyl, nitro, cyano, mercapto or carboxyl group, an alkyl, monohydroxyalkyl, polyhydroxyalkyl, thioalkyl, cyanoalkyl, alkoxy, acyl or acetyloxy group, a cycloalkyl, aryl or alkylaryl group or an -NHR_{N} group in which R_{N} denotes a hydrogen atom or an alkyl, monohydroxyalkyl, polyhydroxyalkyl, acetyl or ureido group;
two of the R₆, R₇ or R₈ radicals can also form, jointly with the atoms to which they are attached, a 5-to 7-membered aromatic ring.

4. Use according to Claim 1 or 2, **characterized in that** the A₁^{⊕} and A₂^{⊕} groups simultaneously denote a quaternary imidazole group of formula (IV) in which R₃, R₄ and R₅, which are identical or different, denote a hydrogen atom, a hydroxyl, nitro, cyano, mercapto or carboxyl group or an alkyl, monohydroxyalkyl or polyhydroxyalkyl group; the R₄ and R₅ radicals can also form, jointly with the atoms to which they are attached, a 5- to 7-membered aromatic ring;
• B₁ and B₂, which are identical or different, denote a polymethylene group which can comprise one or more oxygen atoms and/or one or more aromatic rings;
• X^{⊖} is as defined in Claim 1.

5. Use according to any one of Claims 1, 2 and 4, **characterized in that** A₁^{⊕} and A₂^{⊕} simultaneously denote a quaternary imidazole group of formula (IV) in which:
• R₃, R₄ and R₅, which are identical or different, denote a hydrogen atom or a C₁ to C₆ alkyl group and the R₄ and R₅ radicals can also form, jointly with the atoms to which they are attached, a phenyl ring;
• B₁ and B₂, which are identical or different, denote a polymethylene group which can comprise one or more oxygen atoms and/or one or more aromatic rings;
• X^{⊖} is as defined in Claim 1.

6. Use according to Claim 1 or 2, **characterized in that** A₁^{⊕} represents a quaternary ammonium of formula (III) in which R₁ and R₂, which are identical or different, denote a hydrogen atom or a C₁ to C₆ alkyl group or jointly form a cyclopentyl group and A₂^{⊕} denotes a quaternary imidazole group of formula (IV) in which R₃, R₄ and R₅, which are identical or different, denote a hydrogen atom or a C₁-C₆ alkyl and the R₄ and R₅ radicals can also form, jointly with the atoms to which they are attached, a 5- to 7-membered aromatic ring.

7. Use according to any one of Claims 1 to 3, **characterized in that** at least one of the B₁ or B₂ groups denotes a group of formula (VI):
- (CH₂)ₙ-CO-A-OC-(CH₂)ₙ- (VI)
in which:
n denotes an integer from 1 to 10;
A denotes:
(a) a glycol residue of formula: -O-Z-O- in which Z denotes a hydrocarbonaceous group or a group corresponding to the formulae: 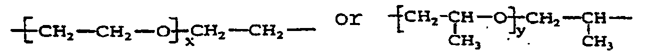 in which x and y denote any number from 1 to 4; or
(b) a bis(secondary amine) residue; or
(c) a bis(primary amine) residue of formula:
-NH-Y-NH-
in which Y denotes a hydrocarbonaceous group or the radical
-(CH₂)₂-S-S-(CH₂)₂- ;
or
(d) a ureylene group.

8. Use according to any one of Claims 1 to 7, **characterized in that** B₁ and B₂, which are identical or different, denote a group of formula:
―(CH₂)ₙ―,
n denoting an integer from 1 to 6; or 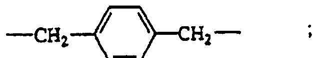 or
― (CH₂)₂―O―(CH₂)₂―O―(CH₂)₂―.

9. Use according to any one of Claims 1 to 8, **characterized in that** X^{⊖} represents a halide anion.

10. Polymers basically composed of repeat units of formula (VII): 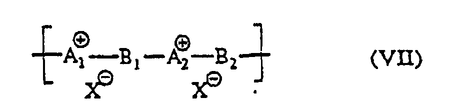 in which
A₁^{⊕} denotes an unsaturated cationic group of formula (II) and
A₂^{⊕} denotes a quaternary ammonium of formula (III);
B₁, B₂, X^{⊖} and the formulae (II) and (III) being as defined in Claim 1 and at least one of the B₁ or B₂ groups being other than -CH₂-CHOH-CH₂-.

11. Polymers basically composed of repeat units of formula (VIII): 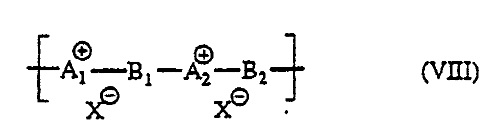 in which:
A₁^{⊕} denotes a quaternary imidazole group of formula (IV) and A₂^{⊕} denotes a quaternary pyrazole group of formula (V) ; B₁, B₂, X^{⊖} and the formulae (IV) and (V) being as defined in Claims 1, 2 and 3.

12. Polymers basically composed of repeat units of formula (IX): 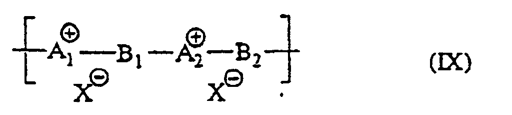 in which:
A₁^{⊕} is as defined in Claim 1 and in which A₂^{⊕} denotes a quaternary imidazole group of formula (IV) as defined in Claim 2 in which R₄ and R₅, which are identical or different, denote a hydrogen or halogen atom, a hydroxyl, nitro, cyano, mercapto or carboxyl group, an alkyl, monohydroxyalkyl, polyhydroxyalkyl, thioalkyl, cyanoalkyl, alkoxy, acyl or acetyloxy group, a cycloalkyl, aryl or alkylaryl group or an -NHR_{N} group in which R_{N} denotes a hydrogen atom or an alkyl, monohydroxyalkyl, polyhydroxyalkyl, acetyl or ureido group and R₃ denotes a cycloalkyl, aryl or alkylaryl group; B₁, B₂ and X^{⊖} being as defined in Claim 1.

13. Reducing composition for the permanent shaping of keratinous fibres, **characterized in that** it comprises, in a medium appropriate for permanent waving, at least one reducing agent and at least one polymer as defined in any one of Claims 1 to 12.

14. Composition according to Claim 13, **characterized in that** the reducing agent is chosen from sulphites, bisulphites and thiols.

15. Reducing composition according to Claim 13 or 14, **characterized in that** the reducing agents are chosen from cysteine, cysteamine and their cosmetically acceptable salts or thiolactic acid, thioglycolic acid and their cosmetically acceptable esters.

16. Reducing composition according to any one of Claims 13 to 15, **characterized in that** it comprises 0.01 to 10% by weight of polymer as defined in any one of Claims 1 to 12.

17. Oxidizing composition for the permanent shaping of keratinous fibres, **characterized in that** it comprises, in a medium appropriate for permanent waving, at least one oxidizing agent and at least one polymer as defined in any one of Claims 1 to 12.

18. Oxidizing composition according to Claim 17, **characterized in that** the oxidizing agent is chosen from aqueous hydrogen peroxide solution, urea hydrogen peroxide, bromates, such as alkaline bromates, persalts or a mixture of alkaline bromates and of a persalt.

19. Oxidizing composition according to Claim 17 or 18, **characterized in that** it comprises from 0.01 to 10% by weight of a polymer as defined in any one of Claims 1 to 12.

20. Composition for the direct dyeing of keratinous fibres and in particular of human keratinous fibres, such as the hair, **characterized in that** it comprises, in a medium appropriate for dyeing, at least one polymer as defined in any one of Claims 1 to 12 and at least one direct dye.

21. Composition according to Claim 20, **characterized in that** the direct dye is chosen from azo dyes, anthraquinone dyes and nitro derivatives of the benzene series.

22. Composition for the oxidation dyeing of keratinous fibres, in particular of human keratinous fibres, such as the hair, **characterized in that** it comprises, in a medium appropriate for dyeing, at least one polymer as defined in any one of Claims 1 to 12 and at least one oxidation dye precursor and/or one melanin precursor.

23. Composition according to Claim 22, **characterized in that** the oxidation dye precursors are chosen from para-phenylenediamines, para-aminophenols, ortho-phenylenediamines and heterocyclic bases.

24. Composition according to Claim 22 or 23, **characterized in that** it additionally comprises couplers.

25. Composition according to Claim 24, **characterized in that** the couplers are chosen from meta-phenylenediamines, meta-aminophenols, meta-diphenols, heterocyclic couplers, indoline derivatives, benzimidazole derivatives, benzomorpholine derivatives, sesamol derivatives, pyridine, pyrimidine and pyrazole derivatives and their addition salts with an acid.

26. Dyeing composition according to any one of Claims 20 to 25, **characterized in that** it comprises from 0.01% to 10% by weight of a polymer as defined in any one of Claims 1 to 12.

27. Oxidizing composition for the oxidation dyeing of keratinous fibres, **characterized in that** it comprises, in a medium appropriate for dyeing, at least one oxidizing agent and at least one polymer as defined in any one of Claims 1 to 12.

28. Oxidizing composition according to Claim 27, **characterized in that** the oxidizing agent is chosen from hydrogen peroxide, urea hydrogen peroxide, alkali metal bromates, persalts, such as perborates and persulphates, and enzymes, such as peroxidases and two-electron oxidoreductases.

29. Bleaching composition, **characterized in that** it comprises, in a medium appropriate for bleaching, an agent for bleaching the hair and a polymer as defined in any one of Claims 1 to 12.

30. Composition according to Claim 29, **characterized in that** the bleaching agent is chosen from hydrogen peroxide, persulphates, sodium percarbonate, or perborates.

31. Hair dyeing process, **characterized in that** a composition comprising a dye precursor, which composition is diluted at the time of use with an oxidizing composition, is applied to the hair in an amount sufficient to dye the hair, that the composition is allowed to act for a time of between 5 and 45 minutes approximately and that the hair is rinsed; the composition comprising the precursor and/or the oxidizing composition respectively being as defined in any one of Claims 22 to 26 and as defined in either of Claims 27 and 28.

32. Hair dyeing process, **characterized in that** the composition as defined in Claim 20, 21 or 22 is applied to the hair in an amount sufficient to produce a colouring, that the composition is allowed to act for a time of between 10 and 60 minutes approximately and that the hair is optionally rinsed.

33. Hair bleaching process, **characterized in that** the bleaching composition defined in either one of Claims 29 and 30 is applied to the hair in an amount and for a time sufficient to bleach the hair and that the hair is subsequently rinsed.

34. Process for the permanent shaping of keratinous fibres, **characterized in that** a reducing composition is applied to the keratinous fibres, that, after a setting time sufficient to reduce the keratin, an oxidizing composition is applied and that, after a setting time sufficient to fix the keratinous fibres in a permanent shape, rinsing is carried out; the reducing composition and/or the oxidizing composition respectively being as defined in any one of Claims 13 to 16 and as defined in any one of Claims 17 to 19.

35. Composition intended for the cosmetic treatment of the hair, **characterized in that** it comprises at least one polymer as defined in any one of Claims 10 to 12.
